# EUROPEAN PATENT SPECIFICATION

(11) **EP 2 923 651 B1**
(45) Date of publication and mention of the grant of the patent: **24.04.2019**
(21) Application number: 15161160.5
(22) Date of filing: 26.03.2015
(51) Int. Cl.: A61B 17/068

(54) **SURGICAL INSTRUMENT UTILIZING SENSOR ADAPTATION**
CHIRURGISCHES INSTRUMENT MIT SENSORANPASSUNG
INSTRUMENT CHIRURGICAL UTILISANT UNE ADAPTATION DE CAPTEUR

(30) Priority: 26.03.2014 US 201414226116
(43) Date of publication of application: 30.09.2015
(73) Proprietor: Ethicon LLC, 00969 Guaynabo (PR)
(72) Inventor: Lytle, IV, Thomas W., Liberty Township, OH Ohio 45044 (US); Overmyer, Mark D., Cincinnati, OH Ohio 45236 (US); Adams, Shane R., Lebanon, OH Ohio 45036 (US); Leimbach, Richard L., Cincinnati, OH Ohio 45209 (US); Shelton, IV, Frederick E., Hillsboro, OH Ohio 45133 (US); Swensgard, Brett E., West Chester, OH Ohio 45069 (US); Houser, Kevin L., Springboro, OH Ohio 45066 (US)
(74) Representative: Carpmaels & Ransford LLP

(56) References cited:
- EP-A1- 1 813 201
- US-A- 5 712 460
- US-A1- 2010 061 181

## Description

### BACKGROUND

The present invention relates to surgical instruments and, in various circumstances, to surgical stapling and cutting instruments and staple cartridges therefor that are designed to staple and cut tissue. A multi-function surgical device control system is known from US5712460A. A motorized bone cement mixer capable of monitoring the state of the cement as it is mixed is known from US2010061181A1. A surgical instrument having recording capabilities is known from EP1813201A1.

### BRIEF DESCRIPTION OF THE DRAWINGS

The features and advantages of this invention, and the manner of attaining them, will become more apparent and the invention itself will be better understood by reference to the following description of embodiments of the invention taken in conjunction with the accompanying drawings, wherein:
FIG. 1 is a perspective view of a surgical instrument that has an interchangeable shaft assembly operably coupled thereto;
FIG. 2 is an exploded assembly view of the interchangeable shaft assembly and surgical instrument of FIG. 1;
FIG. 3 is another exploded assembly view showing portions of the interchangeable shaft assembly and surgical instrument of FIGS. 1 and 2;
FIG. 4 is an exploded assembly view of a portion of the surgical instrument of FIGS. 1-3;
FIG. 5 is a cross-sectional side view of a portion of the surgical instrument of FIG. 4 with the firing trigger in a fully actuated position;
FIG. 6 is another cross-sectional view of a portion of the surgical instrument of FIG. 5 with the firing trigger in an unactuated position;
FIG. 7 is an exploded assembly view of one form of an interchangeable shaft assembly;
FIG. 8 is another exploded assembly view of portions of the interchangeable shaft assembly of FIG. 7;
FIG. 9 is another exploded assembly view of portions of the interchangeable shaft assembly of FIGS. 7 and 8;
FIG. 10 is a cross-sectional view of a portion of the interchangeable shaft assembly of FIGS. 7-9;
FIG. 11 is a perspective view of a portion of the shaft assembly of FIGS. 7-10 with the switch drum omitted for clarity;
FIG. 12 is another perspective view of the portion of the interchangeable shaft assembly of FIG. 11 with the switch drum mounted thereon;
FIG. 13 is a perspective view of a portion of the interchangeable shaft assembly of FIG. 11 operably coupled to a portion of the surgical instrument of FIG. 1 illustrated with the closure trigger thereof in an unactuated position;
FIG. 14 is a right side elevational view of the interchangeable shaft assembly and surgical instrument of FIG. 13;
FIG. 15 is a left side elevational view of the interchangeable shaft assembly and surgical instrument of FIGS. 13 and 14;
FIG. 16 is a perspective view of a portion of the interchangeable shaft assembly of FIG. 11 operably coupled to a portion of the surgical instrument of FIG. 1 illustrated with the closure trigger thereof in an actuated position and a firing trigger thereof in an unactuated position;
FIG. 17 is a right side elevational view of the interchangeable shaft assembly and surgical instrument of FIG. 16;
FIG. 18 is a left side elevational view of the interchangeable shaft assembly and surgical instrument of FIGS. 16 and 17;
FIG. 18A is a right side elevational view of the interchangeable shaft assembly of FIG. 11 operably coupled to a portion of the surgical instrument of FIG. 1 illustrated with the closure trigger thereof in an actuated position and the firing trigger thereof in an actuated position;

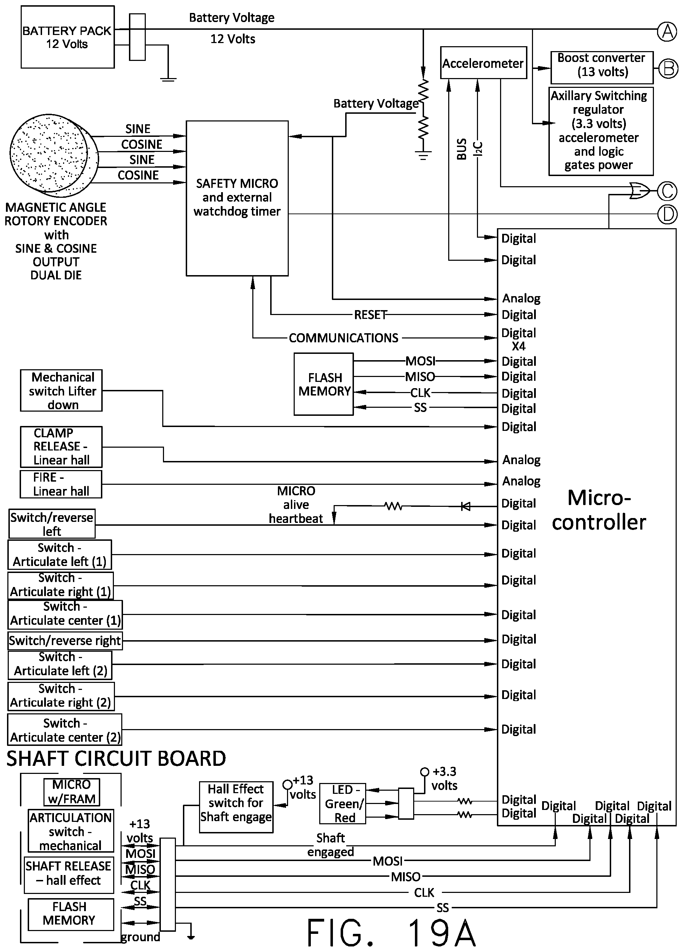

FIG. 19A is a first portion of a schematic for controlling a surgical instrument;

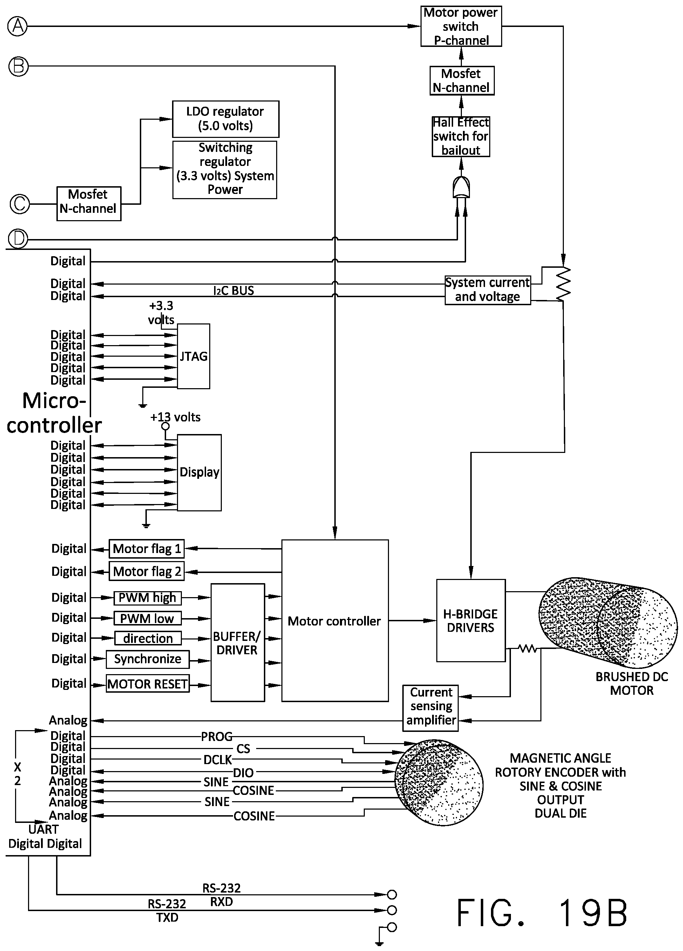

FIG. 19B is a second portion of the schematic of FIG. 19A;

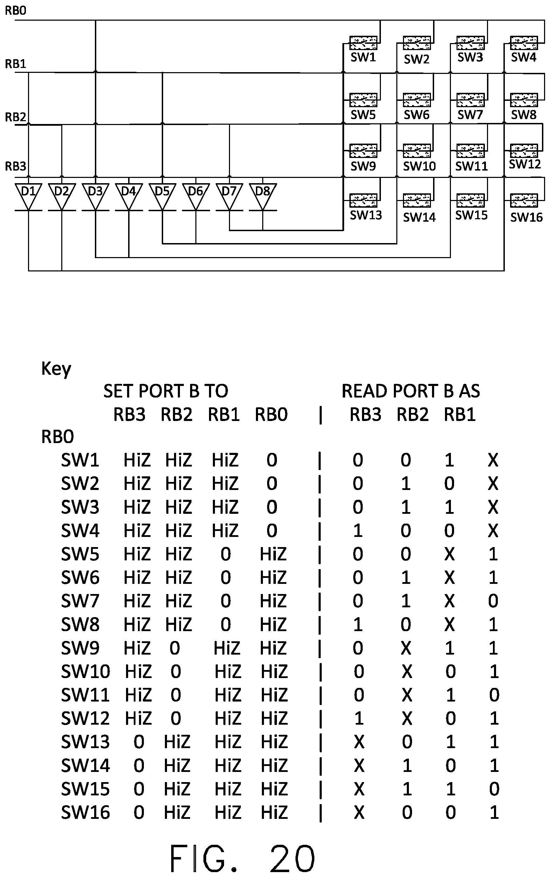

FIG. 20 is a schematic of a switch circuit for use with a surgical instrument;

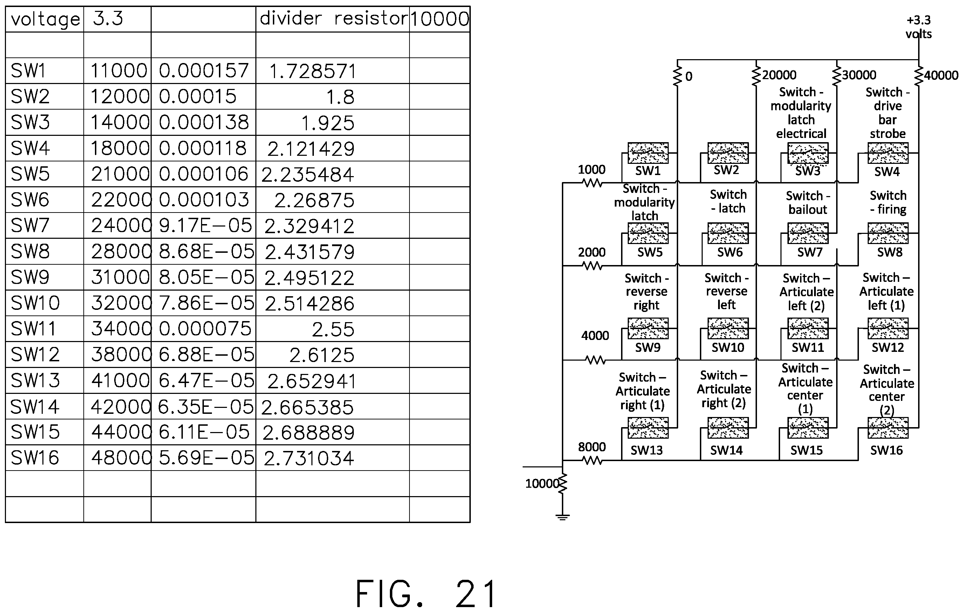

FIG. 21 is another schematic of a switch circuit for use with a surgical instrument;

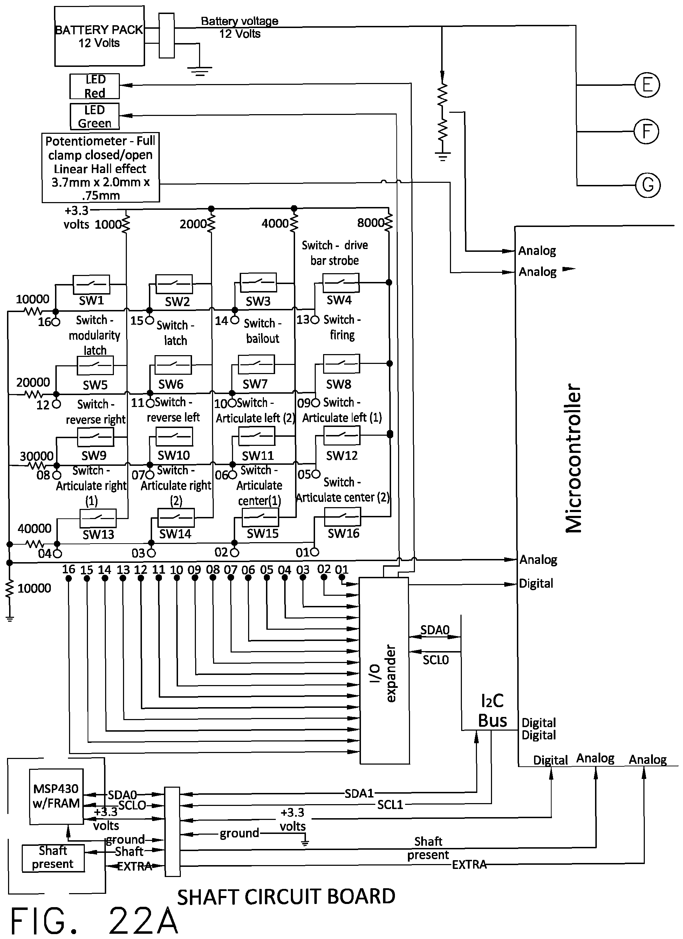

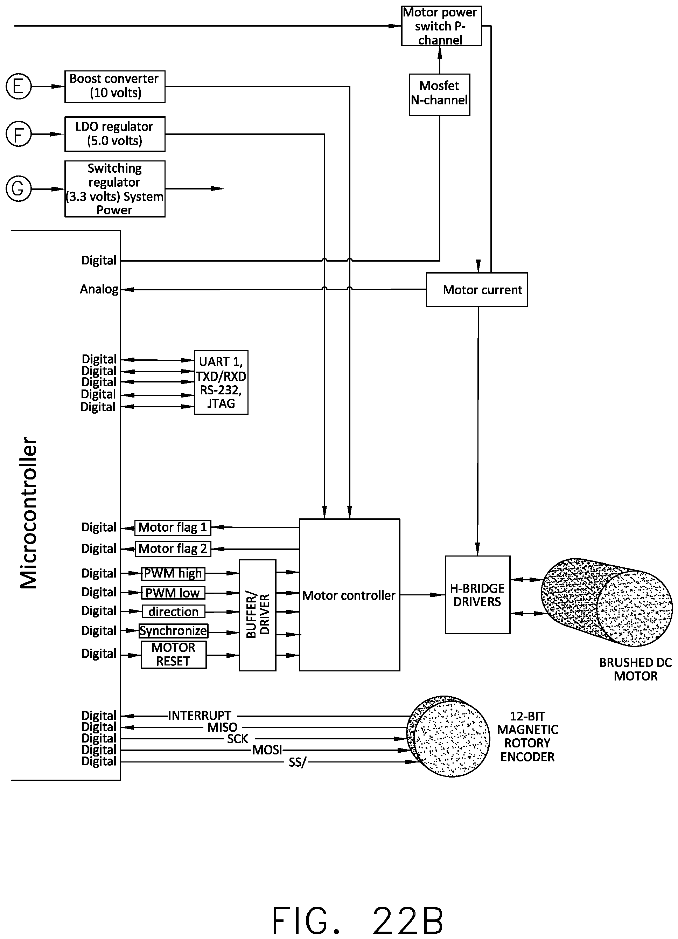

FIG. 22A is a first portion of a schematic for controlling a surgical instrument utilizing the switch circuit of FIG. 21; and FIG. 22B is a second portion of the schematic of FIG. 22A.

### DETAILED DESCRIPTION

Certain exemplary embodiments will now be described to provide an overall understanding of the principles of the structure, function, manufacture, and use of the devices and methods disclosed herein.

The terms "proximal" and "distal" are used herein with reference to a clinician manipulating the handle portion of the surgical instrument. The term "proximal" referring to the portion closest to the clinician and the term "distal" referring to the portion located away from the clinician. It will be further appreciated that, for convenience and clarity, spatial terms such as "vertical," "horizontal," "up," and "down" may be used herein with respect to the drawings. However, surgical instruments are used in many orientations and positions, and these terms are not intended to be limiting and/or absolute.

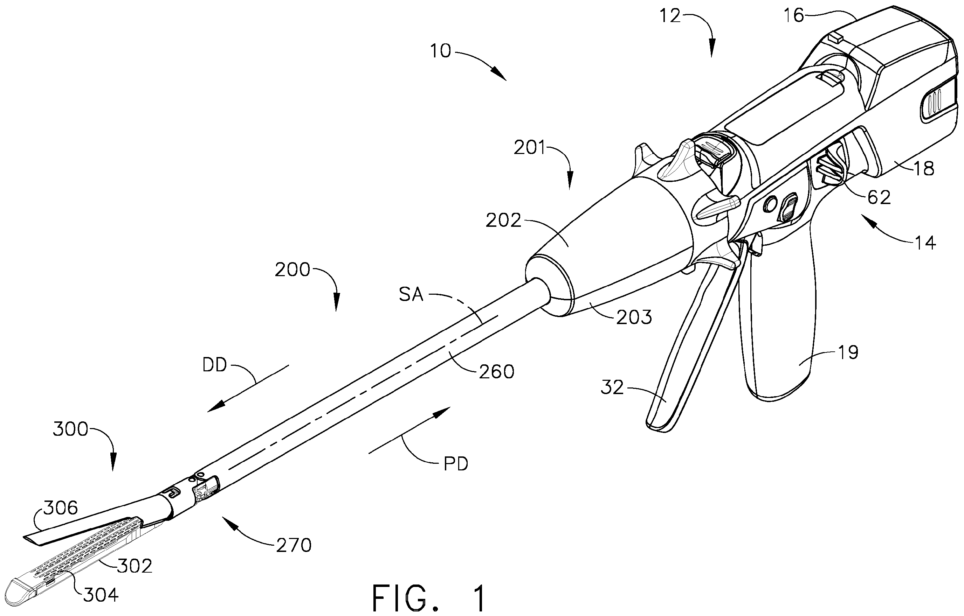

FIGS. 1-6 depict a motor-driven surgical cutting and fastening instrument 10 that may or may not be reused. In the illustrated embodiment, the instrument 10 includes a housing 12 that comprises a handle 14 that is configured to be grasped, manipulated and actuated by the clinician. The housing 12 is configured for operable attachment to an interchangeable shaft assembly 200 that has a surgical end effector 300 operably coupled thereto that is configured to perform one or more surgical tasks or procedures. As the present Detailed Description proceeds, it will be understood that the various unique and novel arrangements of the various forms of interchangeable shaft assemblies disclosed herein may also be effectively employed in connection with robotically-controlled surgical systems. Thus, the term "housing" may also encompass a housing or similar portion of a robotic system that houses or otherwise operably supports at least one drive system that is configured to generate and apply at least one control motion which could be used to actuate the interchangeable shaft assemblies disclosed herein and their respective equivalents. The term "frame" may refer to a portion of a handheld surgical instrument. The term "frame" may also represent a portion of a robotically controlled surgical instrument and/or a portion of the robotic system that may be used to operably control a surgical instrument. For example, the interchangeable shaft assemblies disclosed herein may be employed with various robotic systems, instruments, components and methods disclosed in U.S. Patent Application Serial No. 13/118,241, entitled SURGICAL STAPLING INSTRUMENTS WITH ROTATABLE STAPLE DEPLOYMENT ARRANGEMENTS, now U.S. Patent Application Publication No. US 2012/0298719.

The housing 12 depicted in FIGS. 1-3 is shown in connection with an interchangeable shaft assembly 200 that includes an end effector 300 that comprises a surgical cutting and fastening device that is configured to operably support a surgical staple cartridge 304 therein. The housing 12 may be configured for use in connection with interchangeable shaft assemblies that include end effectors that are adapted to support different sizes and types of staple cartridges, have different shaft lengths, sizes, and types, etc. In addition, the housing 12 may also be effectively employed with a variety of other interchangeable shaft assemblies including those assemblies that are configured to apply other motions and forms of energy such as radio frequency (RF) energy, ultrasonic energy and/or motion to end effector arrangements adapted for use in connection with various surgical applications and procedures. Furthermore, the end effectors, shaft assemblies, handles, surgical instruments, and/or surgical instrument systems can utilize any suitable fastener, or fasteners, to fasten tissue. For instance, a fastener cartridge comprising a plurality of fasteners removably stored therein can be removably inserted into and/or attached to the end effector of a shaft assembly.

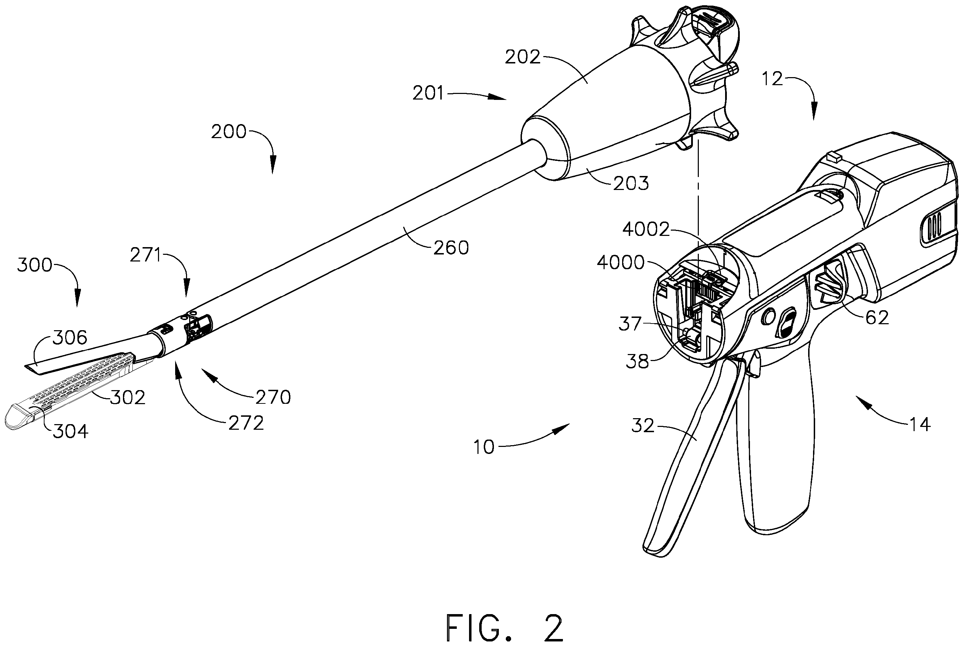

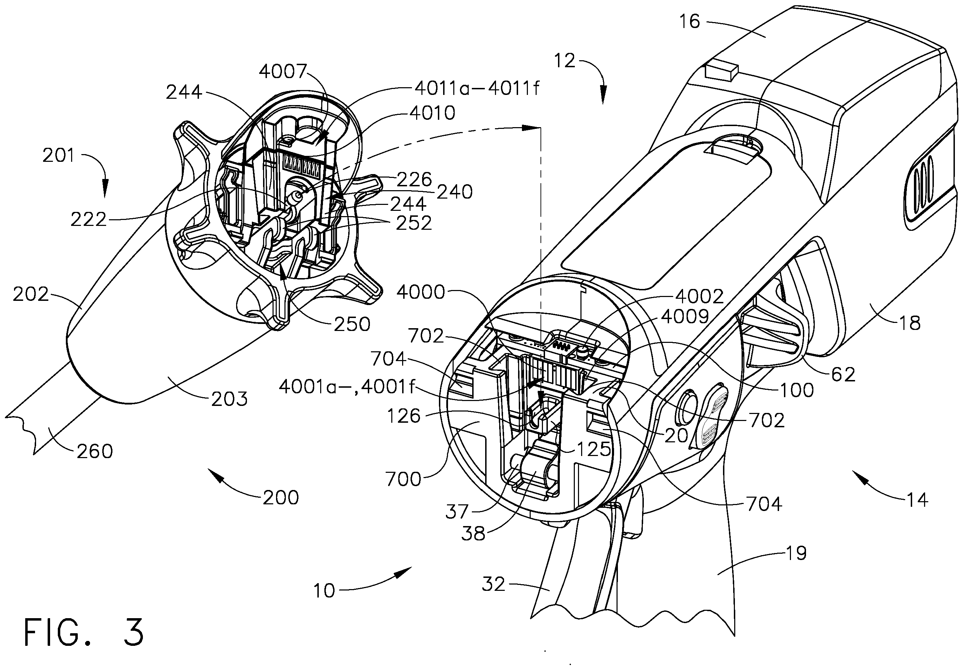

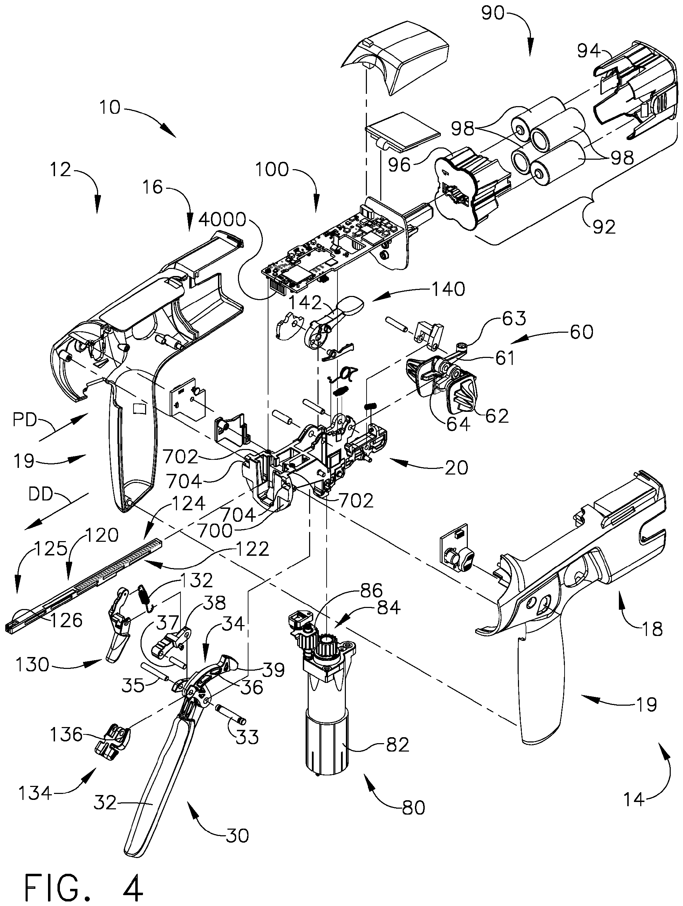

FIG. 1 illustrates the surgical instrument 10 with an interchangeable shaft assembly 200 operably coupled thereto. FIGS. 2 and 3 illustrate attachment of the interchangeable shaft assembly 200 to the housing 12 or handle 14. As can be seen in FIG. 4, the handle 14 comprises a pair of interconnectable handle housing segments 16 and 18 that may be interconnected by screws, snap features, adhesive, etc. In the illustrated arrangement, the handle housing segments 16, 18 cooperate to form a pistol grip portion 19 that can be gripped and manipulated by the clinician. As will be discussed in further detail below, the handle 14 operably supports a plurality of drive systems therein that are configured to generate and apply various control motions to corresponding portions of the interchangeable shaft assembly that is operably attached thereto.

Referring now to FIG. 4, the handle 14 further includes a frame 20 that operably supports a plurality of drive systems. In particular, the frame 20 operably supports a "first" or closure drive system, generally designated as 30, which may be employed to apply closing and opening motions to the interchangeable shaft assembly 200 that is operably attached or coupled thereto. The closure drive system 30 includes an actuator in the form of a closure trigger 32 that is pivotally supported by the frame 20. More specifically, as illustrated in FIG. 4, the closure trigger 32 is pivotally coupled to the housing 14 by a pin 33. Such arrangement enables the closure trigger 32 to be manipulated by a clinician such that when the clinician grips the pistol grip portion 19 of the handle 14, the closure trigger 32 may be easily pivoted from a starting or "unactuated" position to an "actuated" position and more particularly to a fully compressed or fully actuated position. The closure trigger 32 may be biased into the unactuated position by spring or other biasing arrangement (not shown). The closure drive system 30 further includes a closure linkage assembly 34 that is pivotally coupled to the closure trigger 32. As can be seen in FIG. 4, the closure linkage assembly 34 includes a first closure link 36 and a second closure link 38 that are pivotally coupled to the closure trigger 32 by a pin 35. The second closure link 38 may also be referred to herein as an "attachment member" and includes a transverse attachment pin 37.

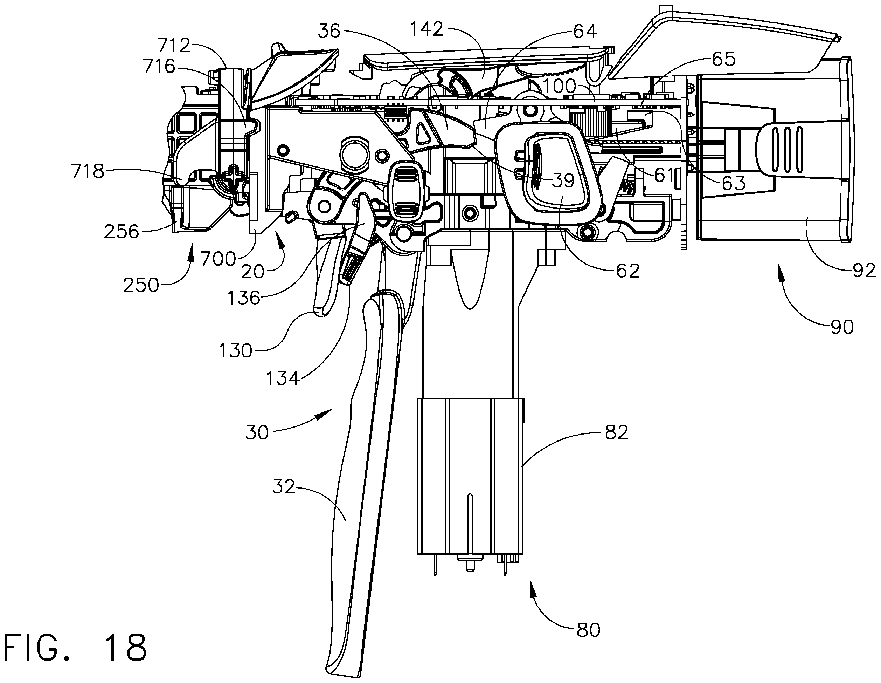

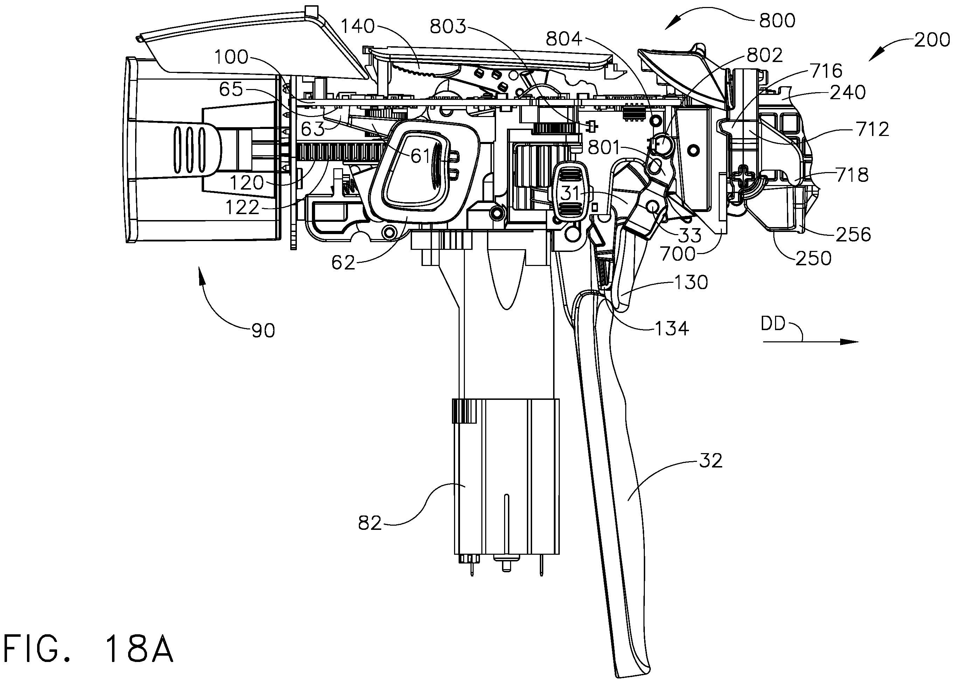

Still referring to FIG. 4, it can be observed that the first closure link 36 has a locking wall or end 39 thereon that is configured to cooperate with a closure release assembly 60 that is pivotally coupled to the frame 20. The closure release assembly 60 comprises a release button assembly 62 that has a distally protruding locking pawl 64 formed thereon. The release button assembly 62 may be pivoted in a counterclockwise direction by a release spring (not shown). As the clinician depresses the closure trigger 32 from its unactuated position towards the pistol grip portion 19 of the handle 14, the first closure link 36 pivots upward to a point wherein the locking pawl 64 drops into retaining engagement with the locking wall 39 on the first closure link 36 thereby preventing the closure trigger 32 from returning to the unactuated position. See FIG. 18. Thus, the closure release assembly 60 serves to lock the closure trigger 32 in the fully actuated position. When the clinician desires to unlock the closure trigger 32 to permit it to be biased to the unactuated position, the clinician simply pivots the closure release button assembly 62 such that the locking pawl 64 is moved out of engagement with the locking wall 39 on the first closure link 36. When the locking pawl 64 has been moved out of engagement with the first closure link 36, the closure trigger 32 may pivot back to the unactuated position. Other closure trigger locking and release arrangements may also be employed.

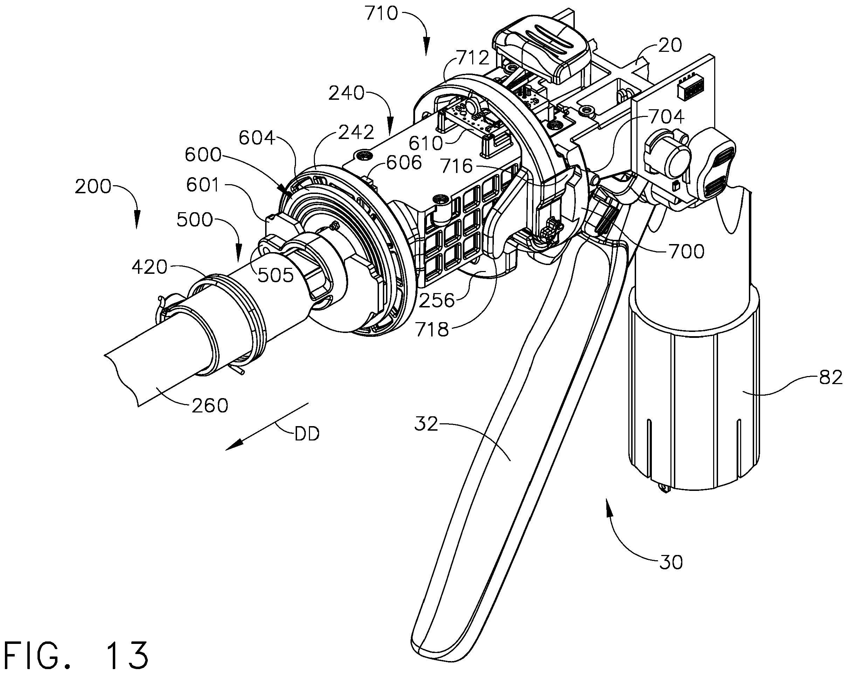

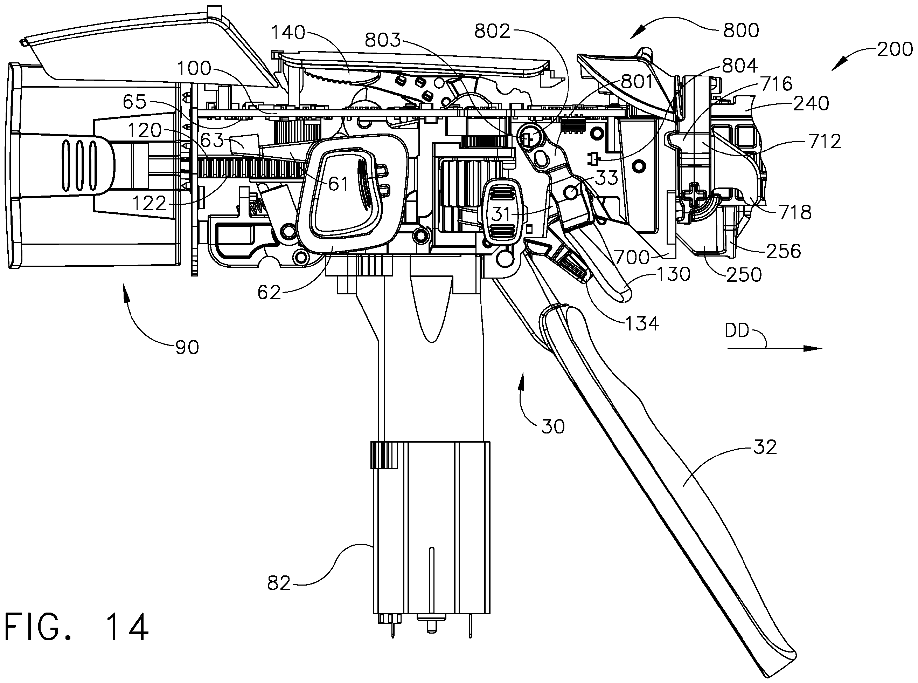

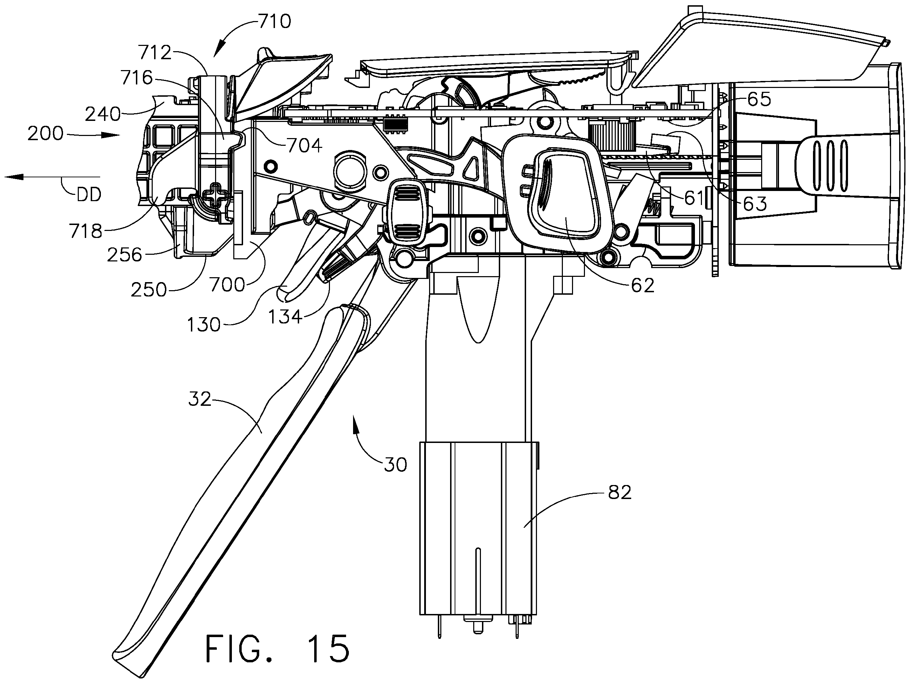

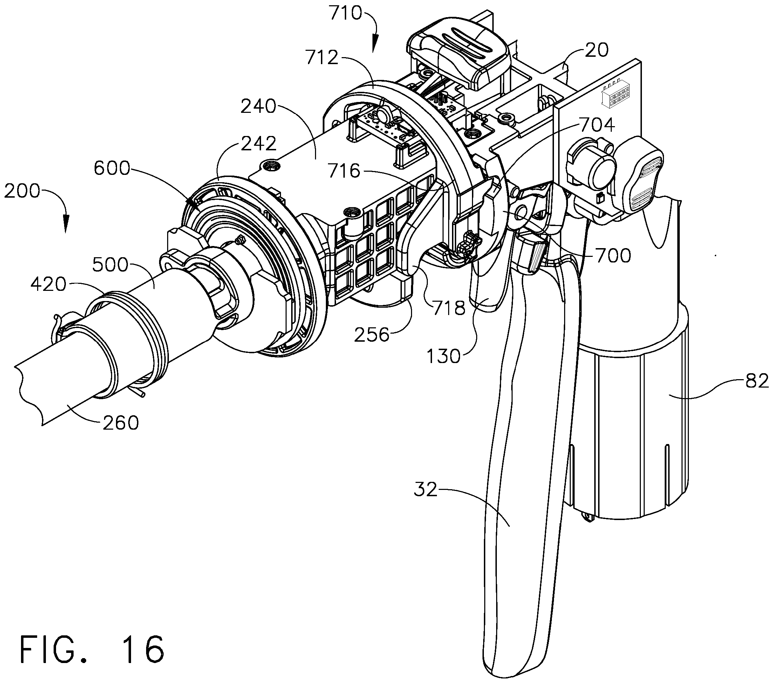

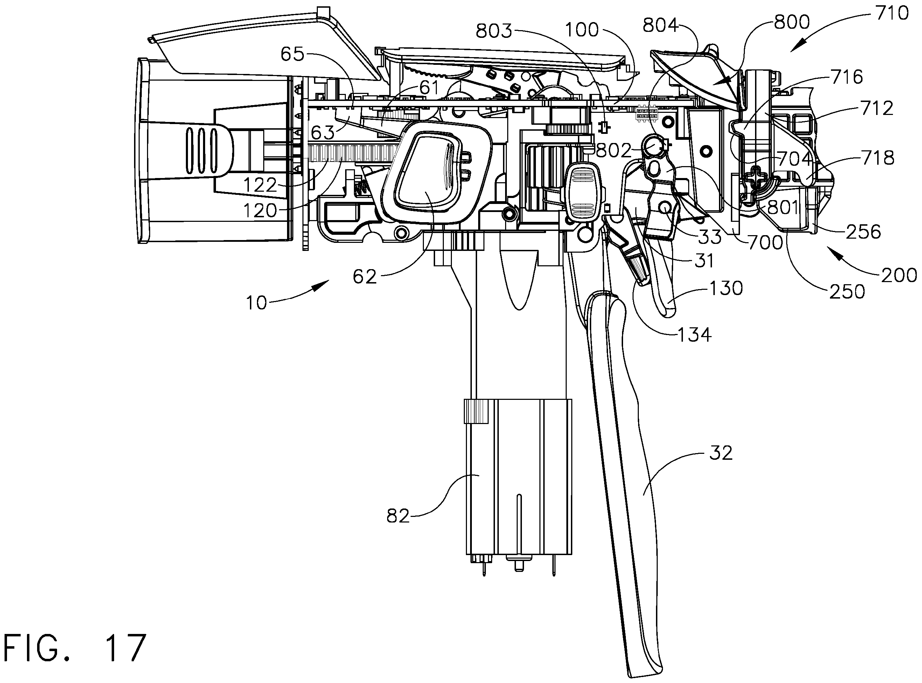

Further to the above, FIGS. 13-15 illustrate the closure trigger 32 in its unactuated position which is associated with an open, or unclamped, configuration of the shaft assembly 200 in which tissue can be positioned between the jaws of the shaft assembly 200. FIGS. 16-18 illustrate the closure trigger 32 in its actuated position which is associated with a closed, or clamped, configuration of the shaft assembly 200 in which tissue is clamped between the jaws of the shaft assembly 200. Upon comparing FIGS. 14 and 17, the reader will appreciate that, when the closure trigger 32 is moved from its unactuated position (FIG. 14) to its actuated position (FIG. 17), the closure release button 62 is pivoted between a first position (FIG. 14) and a second position (FIG. 17). The rotation of the closure release button 62 can be referred to as being an upward rotation; however, at least a portion of the closure release button 62 is being rotated toward the circuit board 100. Referring to FIG. 4, the closure release button 62 can include an arm 61 extending therefrom and a magnetic element 63, such as a permanent magnet mounted to the arm 61. When the closure release button 62 is rotated from its first position to its second position, the magnetic element 63 moves toward the circuit board 100. The circuit board 100 includes at least one sensor configured to detect the movement of the magnetic element 63. A Hall effect sensor 65 can be mounted to the bottom surface of the circuit board 100. The Hall effect sensor 65 is configured to detect changes in a magnetic field surrounding the Hall effect sensor 65 caused by the movement of the magnetic element 63. The Hall effect sensor 65 is in signal communication with a microcontroller 7004 (FIG. 59) which can determine whether the closure release button 62 is in its first position, which is associated with the unactuated position of the closure trigger 32 and the open configuration of the end effector, its second position, which is associated with the actuated position of the closure trigger 32 and the closed configuration of the end effector, and/or any position between the first position and the second position.

The handle 14 and the frame 20 operably support another drive system referred to herein as a firing drive system 80 that is configured to apply firing motions to corresponding portions of the interchangeable shaft assembly attached thereto. The firing drive system may 80 also be referred to herein as a "second drive system". The firing drive system 80 employs an electric motor 82, located in the pistol grip portion 19 of the handle 14. The motor 82 may be a DC brushed driving motor having a maximum rotation of, approximately, 25,000 RPM . Alternatively, the motor may include a brushless motor, a cordless motor, a synchronous motor, a stepper motor, or any other suitable electric motor. The motor 82 is powered by a power source 90 that may comprise a removable power pack 92. As can be seen in FIG. 4 the power pack 92 may comprise a proximal housing portion 94 that is configured for attachment to a distal housing portion 96. The proximal housing portion 94 and the distal housing portion 96 are configured to operably support a plurality of batteries 98 therein. Batteries 98 may each comprise a Lithium Ion ("LI") or other suitable battery. The distal housing portion 96 is configured for removable operable attachment to a control circuit board assembly 100 which is also operably coupled to the motor 82. A number of batteries 98 may be connected in series may be used as the power source for the surgical instrument 10. In addition, the power source 90 may be replaceable and/or rechargeable.

The electric motor 82 includes a rotatable shaft (not shown) that operably interfaces with a gear reducer assembly 84 that is mounted in meshing engagement with a with a set, or rack, of drive teeth 122 on a longitudinally-movable drive member 120. In use, a voltage polarity provided by the power source 90 operates the electric motor 82 in a clockwise direction wherein the voltage polarity applied to the electric motor by the battery can be reversed in order to operate the electric motor 82 in a counter-clockwise direction. When the electric motor 82 is rotated in one direction, the drive member 120 will be axially driven in the distal direction "DD". When the motor 82 is driven in the opposite rotary direction, the drive member 120 will be axially driven in a proximal direction "PD". The handle 14 includes a switch which can be configured to reverse the polarity applied to the electric motor 82 by the power source 90. The handle 14 can also include a sensor that is configured to detect the position of the drive member 120 and/or the direction in which the drive member 120 is being moved.

Actuation of the motor 82 is controlled by a firing trigger 130 that is pivotally supported on the handle 14. The firing trigger 130 can be pivoted between an unactuated position and an actuated position. The firing trigger 130 is biased into the unactuated position by a spring 132 or other biasing arrangement such that when the clinician releases the firing trigger 130, it is pivoted or otherwise returned to the unactuated position by the spring 132 or biasing arrangement. The firing trigger 130 is positioned "outboard" of the closure trigger 32 as was discussed above. A firing trigger safety button 134 is pivotally mounted to the closure trigger 32 by pin 35. The safety button 134 is positioned between the firing trigger 130 and the closure trigger 32 and has a pivot arm 136 protruding therefrom. See FIG. 4. When the closure trigger 32 is in the unactuated position, the safety button 134 is contained in the handle 14 where the clinician cannot readily access it and move it between a safety position preventing actuation of the firing trigger 130 and a firing position wherein the firing trigger 130 can be fired. As the clinician depresses the closure trigger 32, the safety button 134 and the firing trigger 130 pivot down wherein they can then be manipulated by the clinician.

As discussed above, the handle 14 includes a closure trigger 32 and a firing trigger 130. Referring to FIGS. 14-18A, the firing trigger 130 is pivotably mounted to the closure trigger 32. The closure trigger 32 includes an arm 31 extending therefrom and the firing trigger 130 is pivotably mounted to the arm 31 about a pivot pin 33. When the closure trigger 32 is moved from its unactuated position (FIG. 14) to its actuated position (FIG. 17), the firing trigger 130 descends downwardly, as outlined above. After the safety button 134 has been moved to its firing position, referring primarily to FIG. 18A, the firing trigger 130 can be depressed to operate the motor of the surgical instrument firing system. The handle 14 can include a tracking system, such as system 800 configured to determine the position of the closure trigger 32 and/or the position of the firing trigger 130. With primary reference to FIGS. 14, 17, and 18A, an exemplary tracking system 800 includes a magnetic element, such as permanent magnet 802which is mounted to an arm 801 extending from the firing trigger 130. The tracking system 800 comprises one or more sensors, such as a first Hall effect sensor 803 and a second Hall effect sensor 804which are configured to track the position of the magnet 802. Upon comparing FIGS. 14 and 17, the reader will appreciate that, when the closure trigger 32 is moved from its unactuated position to its actuated position, the magnet 802 moves between a first position adjacent the first Hall effect sensor 803 and a second position adjacent the second Hall effect sensor 804. Upon comparing FIGS. 17 and 18A, the reader will further appreciate that, when the firing trigger 130 is moved from an unfired position (FIG. 17) to a fired position (FIG. 18A), the magnet 802 moves relative to the second Hall effect sensor 804. The sensors 803 and 804 track the movement of the magnet 802 and are in signal communication with a microcontroller on the circuit board 100. With data from the first sensor 803 and/or the second sensor 804, the microcontroller can determine the position of the magnet 802 along a predefined path and, based on that position, the microcontroller can determine whether the closure trigger 32 is in its unactuated position, its actuated position, or a position therebetween. Similarly, with data from the first sensor 803 and/or the second sensor 804, the microcontroller can determine the position of the magnet 802 along a predefined path and, based on that position, the microcontroller can determine whether the firing trigger 130 is in its unfired position, its fully fired position, or a position therebetween.

The longitudinally movable drive member 120 has a rack of teeth 122 formed thereon for meshing engagement with a corresponding drive gear 86 of the gear reducer assembly 84. Optionally, a manually-actuatable "bailout" assembly 140 is configured to enable the clinician to manually retract the longitudinally movable drive member 120 should the motor 82 become disabled. An exemplary bailout assembly 140 includes a lever or bailout handle assembly 142 that is configured to be manually pivoted into ratcheting engagement with teeth 124 also provided in the drive member 120. Thus, the clinician can manually retract the drive member 120 by using the bailout handle assembly 142 to ratchet the drive member 120 in the proximal direction "PD". U.S. Patent Application Publication No. US 2010/0089970 discloses bailout arrangements and other components, arrangements and systems that may also be employed with the instruments disclosed herein.

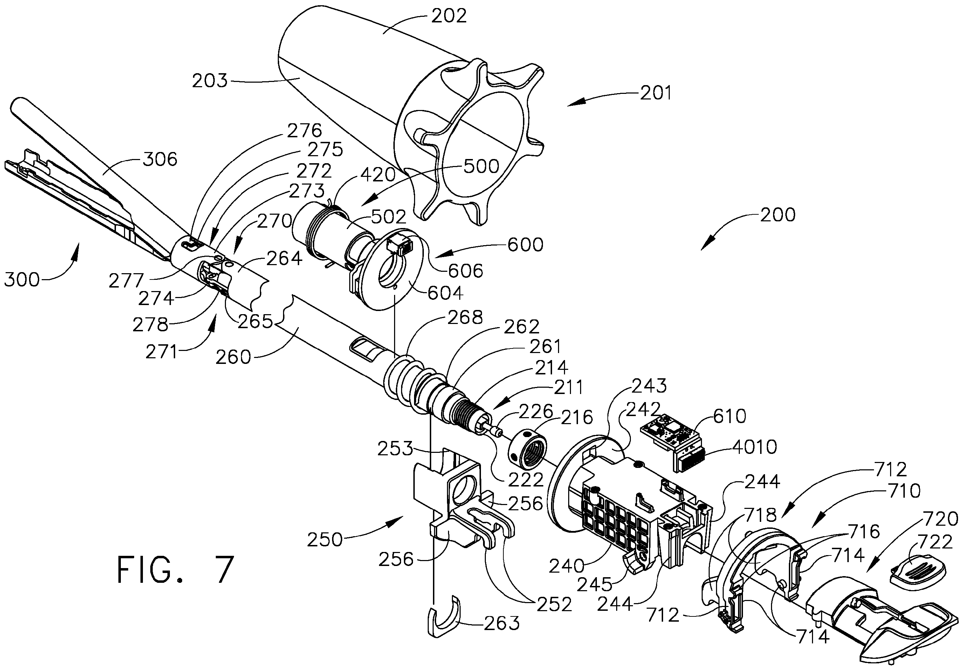

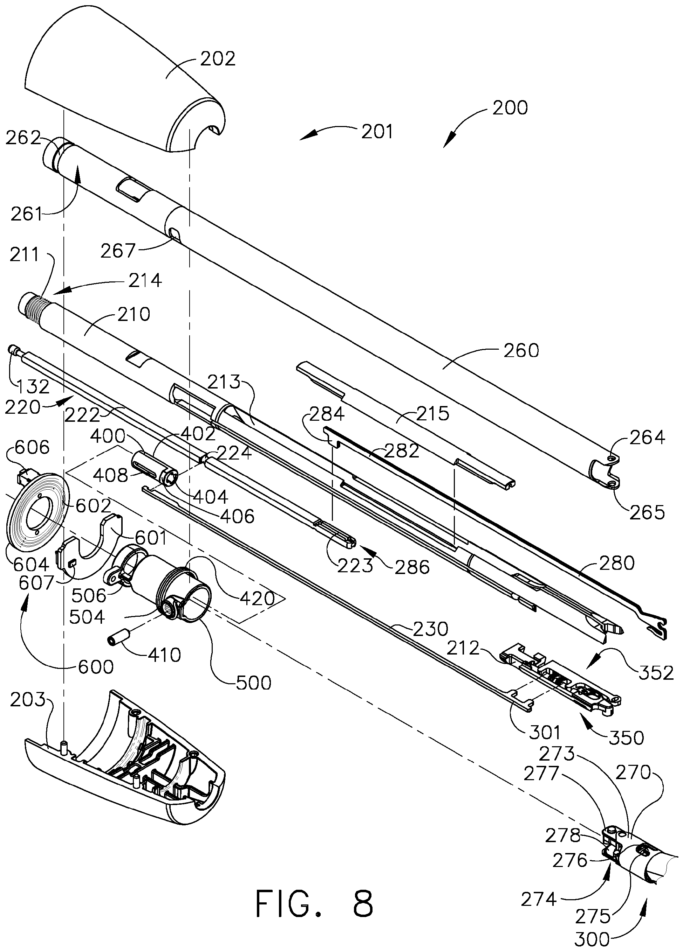

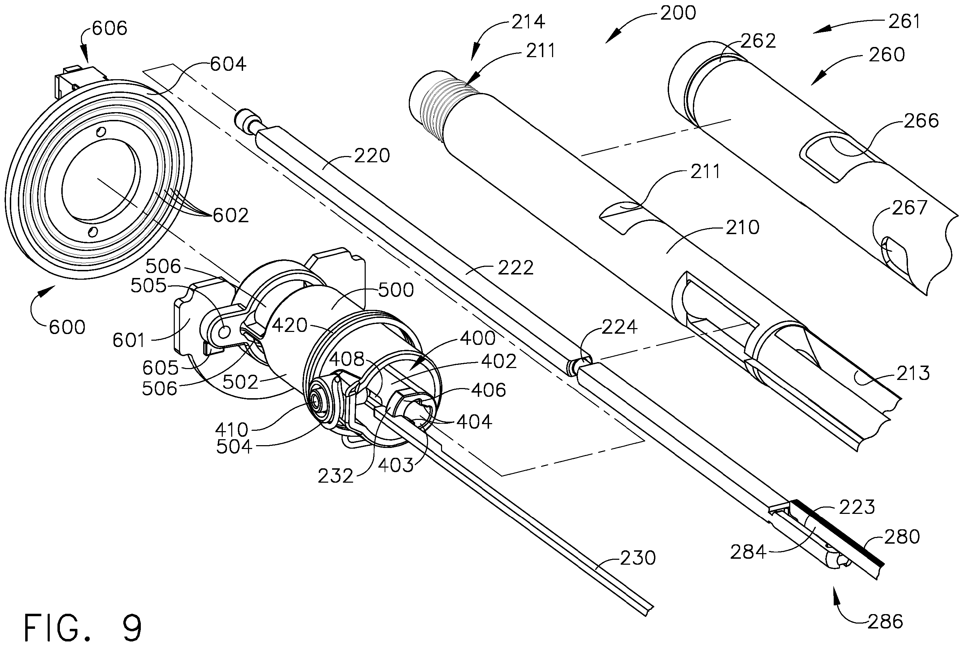

Turning now to FIGS. 1 and 7, the interchangeable shaft assembly 200 includes a surgical end effector 300 that comprises an elongated channel 302 that is configured to operably support a staple cartridge 304 therein. The end effector 300 further includes an anvil 306 that is pivotally supported relative to the elongated channel 302. The interchangeable shaft assembly 200 further includes an articulation joint 270 and an articulation lock 350 (FIG. 8) which is configured to releasably hold the end effector 300 in a desired position relative to a shaft axis SA-SA. Details regarding the construction and operation of the end effector 300, the articulation joint 270 and the articulation lock 350 are set forth in U.S. Patent Application Serial No. 13/803,086, filed March 14, 2013, entitled ARTICULATABLE SURGICAL INSTRUMENT COMPRISING AN ARTICULATION LOCK. As can be seen in FIGS. 7 and 8, the interchangeable shaft assembly 200 further includes a proximal housing or nozzle 201 comprised of nozzle portions 202 and 203. The interchangeable shaft assembly 200 further includes a closure tube 260 which is utilized to close and/or open the anvil 306 of the end effector 300. Primarily referring now to FIGS. 8 and 9, the shaft assembly 200 includes a spine 210 which can be configured to fixably support a shaft frame portion 212 of the articulation lock 350. See FIG. 8. The spine 210 is configured to, one, slidably support a firing member 220 therein and, two, slidably support the closure tube 260 which extends around the spine 210. The spine 210 is also configured to slidably support a proximal articulation driver 230. The articulation driver 230 has a distal end 231 that is configured to operably engage the articulation lock 350. The articulation lock 350 interfaces with an articulation frame 352 that is adapted to operably engage a drive pin (not shown) on the end effector frame (not shown). As indicated above, further details regarding the operation of the articulation lock 350 and the articulation frame may be found in U.S. Patent Application Serial No. 13/803,086. The spine 210 comprises a proximal end 211 which is rotatably supported in a chassis 240. For example, the proximal end 211 of the spine 210 has a thread 214 formed thereon for threaded attachment to a spine bearing 216 configured to be supported within the chassis 240. See FIG. 7. Such an arrangement facilitates rotatable attachment of the spine 210 to the chassis 240 such that the spine 210 may be selectively rotated about a shaft axis SA-SA relative to the chassis 240.

Referring primarily to FIG. 7, the interchangeable shaft assembly 200 includes a closure shuttle 250 that is slidably supported within the chassis 240 such that it may be axially moved relative thereto. As can be seen in FIGS. 3 and 7, the closure shuttle 250 includes a pair of proximally-protruding hooks 252 that are configured for attachment to the attachment pin 37 that is attached to the second closure link 38 as will be discussed in further detail below. A proximal end 261 of the closure tube 260 is coupled to the closure shuttle 250 for relative rotation thereto. For example, a U shaped connector 263 is inserted into an annular slot 262 in the proximal end 261 of the closure tube 260 and is retained within vertical slots 253 in the closure shuttle 250. See FIG. 7. Such an arrangement serves to attach the closure tube 260 to the closure shuttle 250 for axial travel therewith while enabling the closure tube 260 to rotate relative to the closure shuttle 250 about the shaft axis SA-SA. A closure spring 268 is journaled on the closure tube 260 and serves to bias the closure tube 260 in the proximal direction "PD" which can serve to pivot the closure trigger into the unactuated position when the shaft assembly is operably coupled to the handle 14.

The interchangeable shaft assembly 200 further includes an articulation joint 270. Other interchangeable shaft assemblies, however, may be used that are not capable of articulation. As can be seen in FIG. 7 the articulation joint 270 includes a double pivot closure sleeve assembly 271. The double pivot closure sleeve assembly 271 includes an end effector closure sleeve assembly 272 having upper and lower distally projecting tangs 273, 274. An end effector closure sleeve assembly 272 includes a horseshoe aperture 275 and a tab 276 for engaging an opening tab on the anvil 306 in the various manners described in U.S. Patent Application Serial No. 13/803,086, filed March 14, 2013, entitled ARTICULATABLE SURGICAL INSTRUMENT COMPRISING AN ARTICULATION LOCK. As described in further detail therein, the horseshoe aperture 275 and tab 276 engage a tab on the anvil when the anvil 306 is opened. An upper double pivot link 277 includes upwardly projecting distal and proximal pivot pins that engage respectively an upper distal pin hole in the upper proximally projecting tang 273 and an upper proximal pin hole in an upper distally projecting tang 264 on the closure tube 260. A lower double pivot link 278 includes upwardly projecting distal and proximal pivot pins that engage respectively a lower distal pin hole in the lower proximally projecting tang 274 and a lower proximal pin hole in the lower distally projecting tang 265. See also FIG. 8.

In use, the closure tube 260 is translated distally (direction "DD") to close the anvil 306 in response to the actuation of the closure trigger 32. The anvil 306 is closed by distally translating the closure tube 260 and thus the shaft closure sleeve assembly 272, causing it to strike a proximal surface on the anvil 360 in the manner described in the aforementioned reference U.S. Patent Application Serial No. 13/803,086. As was also described in detail in that reference, the anvil 306 is opened by proximally translating the closure tube 260 and the shaft closure sleeve assembly 272, causing tab 276 and the horseshoe aperture 275 to contact and push against the anvil tab to lift the anvil 306. In the anvil-open position, the shaft closure tube 260 is moved to its proximal position.

As indicated above, the surgical instrument 10 may further include an articulation lock 350 of the types and construction described in further detail in U.S. Patent Application Serial No. 13/803,086 which is configured and operated to selectively lock the end effector 300 in position. Such arrangement enables the end effector 300 to be rotated, or articulated, relative to the shaft closure tube 260 when the articulation lock 350 is in its unlocked state. In such an unlocked state, the end effector 300 can be positioned and pushed against soft tissue and/or bone surrounding the surgical site within the patient in order to cause the end effector 300 to articulate relative to the closure tube 260. The end effector 300 may also be articulated relative to the closure tube 260 by an articulation driver 230.

The interchangeable shaft assembly 200 further includes a firing member 220 that is supported for axial travel within the shaft spine 210. The firing member 220 includes an intermediate firing shaft portion 222 that is configured for attachment to a distal cutting portion or knife bar 280. The firing member 220 may also be referred to herein as a "second shaft" and/or a "second shaft assembly". As can be seen in FIGS. 8 and 9, the intermediate firing shaft portion 222 includes a longitudinal slot 223 in the distal end thereof which is configured to receive a tab 284 on the proximal end 282 of the distal knife bar 280. The longitudinal slot 223 and the proximal end 282 are sized and configured to permit relative movement therebetween and comprise a slip joint 286. The slip joint 286 permits the intermediate firing shaft portion 222 of the firing drive 220 to be moved to articulate the end effector 300 without moving, or at least substantially moving, the knife bar 280. Once the end effector 300 has been suitably oriented, the intermediate firing shaft portion 222 can be advanced distally until a proximal sidewall of the longitudinal slot 223 comes into contact with the tab 284 in order to advance the knife bar 280 and fire the staple cartridge positioned within the channel 302 As can be further seen in FIGS. 8 and 9, the shaft spine 210 has an elongate opening or window 213 therein to facilitate assembly and insertion of the intermediate firing shaft portion 222 into the shaft frame 210. Once the intermediate firing shaft portion 222 has been inserted therein, a top frame segment 215 is engaged with the shaft frame 212 to enclose the intermediate firing shaft portion 222 and knife bar 280 therein. Further description of the operation of the firing member 220 may be found in U.S. Patent Application Serial No. 13/803,086.

The shaft assembly 200 includes a clutch assembly 400 which is configured to selectively and releasably couple the articulation driver 230 to the firing member 220. The clutch assembly 400 includes a lock collar, or sleeve 402, positioned around the firing member 220 wherein the lock sleeve 402 can be rotated between an engaged position in which the lock sleeve 402 couples the articulation driver 360 to the firing member 220 and a disengaged position in which the articulation driver 360 is not operably coupled to the firing member 200. When lock sleeve 402 is in its engaged position, distal movement of the firing member 220 can move the articulation driver 360 distally and, correspondingly, proximal movement of the firing member 220 can move the articulation driver 230 proximally. When lock sleeve 402 is in its disengaged position, movement of the firing member 220 is not transmitted to the articulation driver 230 and, as a result, the firing member 220 can move independently of the articulation driver 230. The articulation driver 230 can be held in position by the articulation lock 350 when the articulation driver 230 is not being moved in the proximal or distal directions by the firing member 220.

Referring primarily to FIG. 9, the lock sleeve 402 comprises a cylindrical, or an at least substantially cylindrical, body including a longitudinal aperture 403 defined therein configured to receive the firing member 220. The lock sleeve 402 comprises diametrically-opposed, inwardly-facing lock protrusions 404 and an outwardly-facing lock member 406. The lock protrusions 404 are configured to be selectively engaged with the firing member 220. More particularly, when the lock sleeve 402 is in its engaged position, the lock protrusions 404 are positioned within a drive notch 224 defined in the firing member 220 such that a distal pushing force and/or a proximal pulling force can be transmitted from the firing member 220 to the lock sleeve 402. When the lock sleeve 402 is in its engaged position, the second lock member 406 is received within a drive notch 232 defined in the articulation driver 230 such that the distal pushing force and/or the proximal pulling force applied to the lock sleeve 402 can be transmitted to the articulation driver 230. In effect, the firing member 220, the lock sleeve 402, and the articulation driver 230 will move together when the lock sleeve 402 is in its engaged position. On the other hand, when the lock sleeve 402 is in its disengaged position, the lock protrusions 404 is not positioned within the drive notch 224 of the firing member 220 and, as a result, a distal pushing force and/or a proximal pulling force is not transmitted from the firing member 220 to the lock sleeve 402. Correspondingly, the distal pushing force and/or the proximal pulling force are not transmitted to the articulation driver 230. In such circumstances, the firing member 220 can be slid proximally and/or distally relative to the lock sleeve 402 and the proximal articulation driver 230.

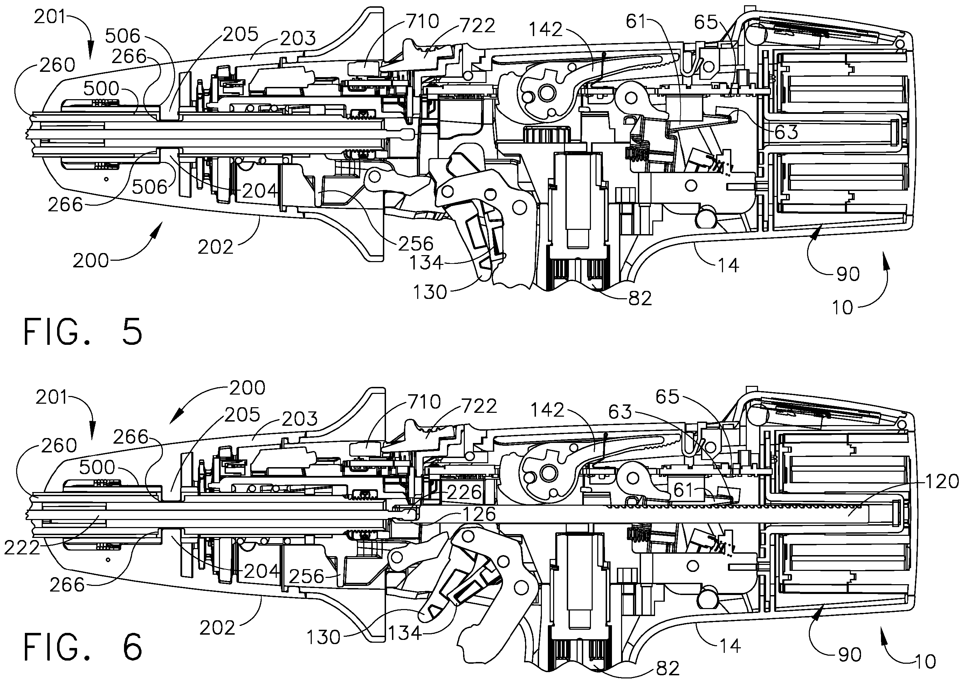

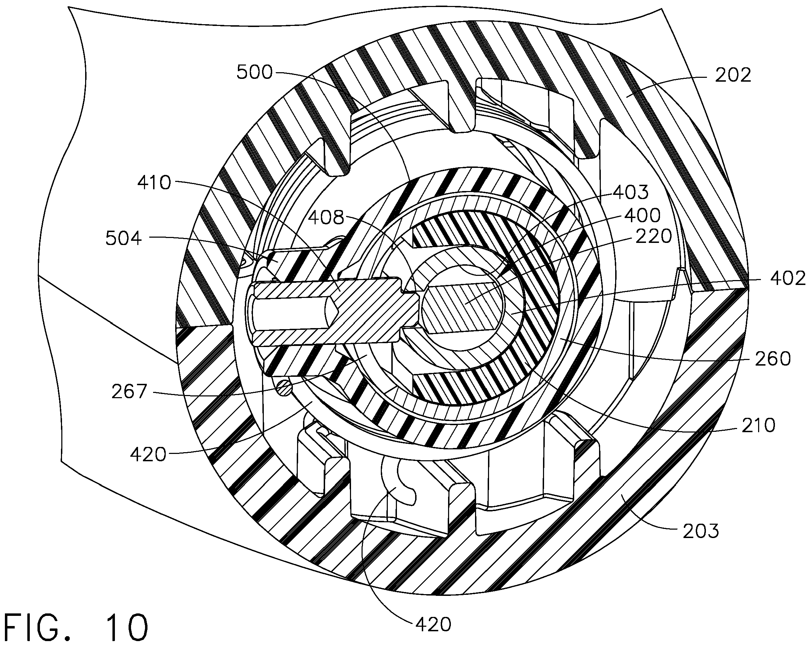

As can be seen in FIGS. 8-12, the shaft assembly 200 further includes a switch drum 500 that is rotatably received on the closure tube 260. The switch drum 500 comprises a hollow shaft segment 502 that has a shaft boss 504 formed thereon for receive an outwardly protruding actuation pin 410 therein. The actuation pin 410 extends through a slot 267 into a longitudinal slot 408 provided in the lock sleeve 402 to facilitate axial movement of the lock sleeve 402 when it is engaged with the articulation driver 230. A rotary torsion spring 420 is configured to engage the boss 504 on the switch drum 500 and a portion of the nozzle housing 203 as shown in FIG. 10 to apply a biasing force to the switch drum 500. The switch drum 500 further comprises at least partially circumferential openings 506 defined therein which, referring to FIGS. 5 and 6, are configured to receive circumferential mounts 204, 205 extending from the nozzle halves 202, 203 and permit relative rotation, but not translation, between the switch drum 500 and the proximal nozzle 201. As can be seen in those Figures, the mounts 204 and 205 also extend through openings 266 in the closure tube 260 to be seated in recesses 211 in the shaft spine 210. However, rotation of the nozzle 201 to a point where the mounts 204, 205 reach the end of their respective slots 506 in the switch drum 500 will result in rotation of the switch drum 500 about the shaft axis SA-SA. Rotation of the switch drum 500 will ultimately result in the rotation of eth actuation pin 410 and the lock sleeve 402 between its engaged and disengaged positions. Thus, in essence, the nozzle 201 may be employed to operably engage and disengage the articulation drive system with the firing drive system in the various manners described in further detail in U.S. Patent Application Serial No. 13/803,086.

As also illustrated in FIGS. 8-12, the shaft assembly 200 comprises a slip ring assembly 600 which is configured to conduct electrical power to and/or from the end effector 300 and/or communicate signals to and/or from the end effector 300. The slip ring assembly 600 comprises a proximal connector flange 604 mounted to a chassis flange 242 extending from the chassis 240 and a distal connector flange 601 positioned within a slot defined in the shaft housings 202, 203. The proximal connector flange 604 comprises a first face and the distal connector flange 601 comprises a second face which is positioned adjacent to and movable relative to the first face. The distal connector flange 601 can rotate relative to the proximal connector flange 604 about the shaft axis SA-SA. The proximal connector flange 604 comprises a plurality of concentric, or at least substantially concentric, conductors 602 defined in the first face thereof. A connector 607 is mounted on the proximal side of the connector flange 601 and has a plurality of contacts (not shown) wherein each contact corresponds to and is in electrical contact with one of the conductors 602. Such an arrangement permits relative rotation between the proximal connector flange 604 and the distal connector flange 601 while maintaining electrical contact therebetween. The proximal connector flange 604 includes an electrical connector 606 which places the conductors 602 in signal communication with a shaft circuit board 610 mounted to the shaft chassis 240 . A wiring harness comprising a plurality of conductors extends between the electrical connector 606 and the shaft circuit board 610. The electrical connector 606 extend proximally through a connector opening 243 defined in the chassis mounting flange 242. See FIG. 7. Further details regarding slip ring assembly 600 may be found in U.S. Patent Application Serial No. 13/803,086.

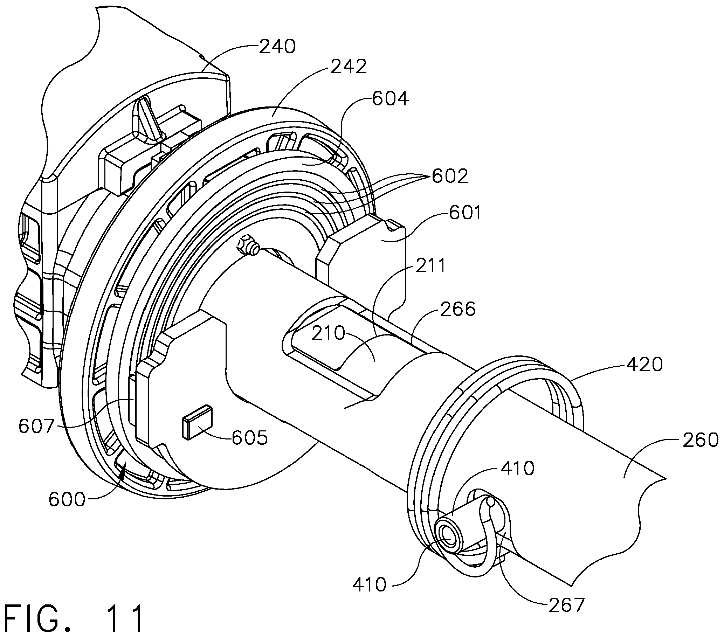

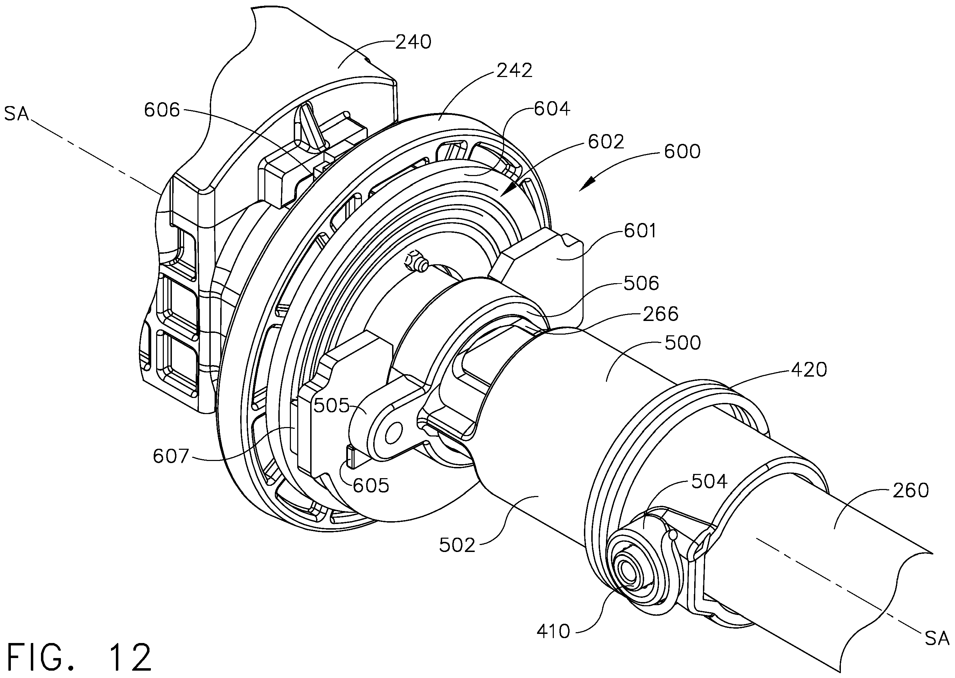

The shaft assembly 200 includes a proximal portion which is fixably mounted to the handle 14 and a distal portion which is rotatable about a longitudinal axis. The rotatable distal shaft portion can be rotated relative to the proximal portion about the slip ring assembly 600, as discussed above. The distal connector flange 601 of the slip ring assembly 600 is positioned within the rotatable distal shaft portion. Moreover, the switch drum 500 is also positioned within the rotatable distal shaft portion. When the rotatable distal shaft portion is rotated, the distal connector flange 601 and the switch drum 500 are rotated synchronously with one another. In addition, the switch drum 500 can be rotated between a first position and a second position relative to the distal connector flange 601. When the switch drum 500 is in its first position, the articulation drive system is operably disengaged from the firing drive system and, thus, the operation of the firing drive system does not articulate the end effector 300 of the shaft assembly 200. When the switch drum 500 is in its second position, the articulation drive system is operably engaged with the firing drive system and, thus, the operation of the firing drive system articulates the end effector 300 of the shaft assembly 200. When the switch drum 500 is moved between its first position and its second position, the switch drum 500 is moved relative to distal connector flange 601. The shaft assembly 200 comprises at least one sensor configured to detect the position of the switch drum 500. Turning now to FIGS. 11 and 12, the distal connector flange 601 comprises a Hall effect sensor 605 and the switch drum 500 comprises a magnetic element, such as permanent magnet 505. The Hall effect sensor 605 is configured to detect the position of the permanent magnet 505. When the switch drum 500 is rotated between its first position and its second position, the permanent magnet 505 moves relative to the Hall effect sensor 605. The Hall effect sensor 605 detects changes in a magnetic field created when the permanent magnet 505 is moved. The Hall effect sensor 605 is in signal communication with the shaft circuit board 610 and/or the handle circuit board 100. Based on the signal from the Hall effect sensor 605, a microcontroller on the shaft circuit board 610 and/or the handle circuit board 100 can determine whether the articulation drive system is engaged with or disengaged from the firing drive system.

Referring again to FIGS. 3 and 7, the chassis 240 includes at least one, and preferably two, tapered attachment portions 244 formed thereon that are adapted to be received within corresponding dovetail slots 702 formed within a distal attachment flange portion 700 of the frame 20. Each dovetail slot 702 is tapered or, stated another way, is somewhat V-shaped to seatingly receive the attachment portions 244 therein. As can be further seen in FIGS. 3 and 7, a shaft attachment lug 226 is formed on the proximal end of the intermediate firing shaft 222. As will be discussed in further detail below, when the interchangeable shaft assembly 200 is coupled to the handle 14, the shaft attachment lug 226 is received in a firing shaft attachment cradle 126 formed in the distal end 125 of the longitudinal drive member 120. See FIGS. 3 and 6.

Various shaft assembly embodiments employ a latch system 710 for removably coupling the shaft assembly 200 to the housing 12 and more specifically to the frame 20. As can be seen in FIG. 7 the latch system 710 includes a lock member or lock yoke 712 that is movably coupled to the chassis 240. In the illustrated embodiment the lock yoke 712 has a U-shape with two spaced downwardly extending legs 714. The legs 714 each have a pivot lug 716 formed thereon that are adapted to be received in corresponding holes 245 formed in the chassis 240. Such arrangement facilitates pivotal attachment of the lock yoke 712 to the chassis 240. The lock yoke 712 includes two proximally protruding lock lugs 714 that are configured for releasable engagement with corresponding lock detents or grooves 704 in the distal attachment flange 700 of the frame 20. See FIG. 3. The lock yoke 712 is biased in the proximal direction by a spring or biasing member (not shown). Actuation of the lock yoke 712 is accomplished by a latch button 722 that is slidably mounted on a latch actuator assembly 720 that is mounted to the chassis 240. The latch button 722 is biased in a proximal direction relative to the lock yoke 712. As will be discussed in further detail below, the lock yoke 712 can be moved to an unlocked position by biasing the latch button the in distal direction which also causes the lock yoke 712 to pivot out of retaining engagement with the distal attachment flange 700 of the frame 20. When the lock yoke 712 is in "retaining engagement" with the distal attachment flange 700 of the frame 20, the lock lugs 716 are retainingly seated within the corresponding lock detents or grooves 704 in the distal attachment flange 700.

When employing an interchangeable shaft assembly that includes an end effector of the type described herein that is adapted to cut and fasten tissue, as well as other types of end effectors, it may be desirable to prevent inadvertent detachment of the interchangeable shaft assembly from the housing during actuation of the end effector. For example, in use the clinician may actuate the closure trigger 32 to grasp and manipulate the target tissue into a desired position. Once the target tissue is positioned within the end effector 300 in a desired orientation, the clinician may then fully actuate the closure trigger 32 to close the anvil 306 and clamp the target tissue in position for cutting and stapling. In that instance, the first drive system 30 has been fully actuated. After the target tissue has been clamped in the end effector 300, it may be desirable to prevent the inadvertent detachment of the shaft assembly 200 from the housing 12. A latch system 710 is configured to prevent such inadvertent detachment.

As can be most particularly seen in FIG. 7, the lock yoke 712 includes at least one and preferably two lock hooks 718 that are adapted to contact corresponding lock lug portions 256 that are formed on the closure shuttle 250. Referring to FIGS. 13-15, when the closure shuttle 250 is in an unactuated position (i.e., the first drive system 30 is unactuated and the anvil 306 is open), the lock yoke 712 may be pivoted in a distal direction to unlock the interchangeable shaft assembly 200 from the housing 12. When in that position, the lock hooks 718 do not contact the lock lug portions 256 on the closure shuttle 250. However, when the closure shuttle 250 is moved to an actuated position (i.e., the first drive system 30 is actuated and the anvil 306 is in the closed position), the lock yoke 712 is prevented from being pivoted to an unlocked position. See FIGS. 16-18. Stated another way, if the clinician were to attempt to pivot the lock yoke 712 to an unlocked position or the lock yoke 712 was in advertently bumped or contacted in a manner that might otherwise cause it to pivot distally, the lock hooks 718 on the lock yoke 712 will contact the lock lugs 256 on the closure shuttle 250 and prevent movement of the lock yoke 712 to an unlocked position.

Attachment of the interchangeable shaft assembly 200 to the handle 14 will now be described with reference to FIG. 3. To commence the coupling process, the clinician positions the chassis 240 of the interchangeable shaft assembly 200 above or adjacent to the distal attachment flange 700 of the frame 20 such that the tapered attachment portions 244 formed on the chassis 240 are aligned with the dovetail slots 702 in the frame 20. The clinician then moves the shaft assembly 200 along an installation axis IA that is perpendicular to the shaft axis SA-SA to seat the attachment portions 244 in "operable engagement" with the corresponding dovetail receiving slots 702. In doing so, the shaft attachment lug 226 on the intermediate firing shaft 222 will also be seated in the cradle 126 in the longitudinally movable drive member 120 and the portions of pin 37 on the second closure link 38 will be seated in the corresponding hooks 252 in the closure yoke 250. As used herein, the term "operable engagement" in the context of two components means that the two components are sufficiently engaged with each other so that upon application of an actuation motion thereto, the components may carry out their intended action, function and/or procedure.

As discussed above, at least five systems of the interchangeable shaft assembly 200 can be operably coupled with at least five corresponding systems of the handle 14. A first system comprises a frame system which couples and/or aligns the frame or spine of the shaft assembly 200 with the frame 20 of the handle 14. Another system comprises a closure drive system 30 which can operably connect the closure trigger 32 of the handle 14 and the closure tube 260 and the anvil 306 of the shaft assembly 200. The closure tube attachment yoke 250 of the shaft assembly 200 is engaged with the pin 37 on the second closure link 38. Another system comprises the firing drive system 80 which operably connects the firing trigger 130 of the handle 14 with the intermediate firing shaft 222 of the shaft assembly 200. As outlined above, the shaft attachment lug 226 is operably connected with the cradle 126 of the longitudinal drive member 120. Another system comprises an electrical system which, one, signals to a controller in the handle 14, such as microcontroller that the shaft assembly 200 has been operably engaged with the handle 14 and/or, two, conducts power and/or communication signals between the shaft assembly 200 and the handle 14. For instance, the shaft assembly 200 can include an electrical connector 4010 that is operably mounted to the shaft circuit board 610. The electrical connector 4010 is configured for mating engagement with a corresponding electrical connector 4000 on the handle control board 100. Further details regaining the circuitry and control systems may be found in U.S. Patent Application Serial No. 13/803,086. The fifth system consists of the latching system for releasably locking the shaft assembly 200 to the handle 14.

As described herein, a surgical instrument, such as a surgical stapling instrument, for example, can include a processor, computer, and/or controller, for example, (herein collectively referred to as a "processor") and one or more sensors in signal communication with the processor, computer, and/or controller. The processor can comprise a microcontroller and one or more memory units operationally coupled to the microcontroller. By executing instruction code stored in the memory, the processor may control various components of the surgical instrument, such as the motor, various drive systems, and/or a user display. The processor may be implemented using integrated and/or discrete hardware elements, software elements, and/or a combination of both.

Examples of integrated hardware elements may include processors, microprocessors, microcontrollers, integrated circuits, application specific integrated circuits (ASIC), programmable logic devices (PLD), digital signal processors (DSP), field programmable gate arrays (FPGA), logic gates, registers, semiconductor devices, chips, microchips, chip sets, microcontrollers, system-on-chip (SoC), and/ or system-in-package (SIP). Examples of discrete hardware elements may include circuits and/or circuit elements such as logic gates, field effect transistors, bipolar transistors, resistors, capacitors, inductors, and/or relays. In certain instances, the processor may include a hybrid circuit comprising discrete and integrated circuit elements or components on one or more substrates.

The processor may be an LM 4F230H5QR, available from Texas Instruments . The Texas Instruments LM4F230H5QR is an ARM Cortex-M4F Processor Core comprising on-chip memory of 256 KB single-cycle flash memory, or other non-volatile memory, up to 40 MHz, a prefetch buffer to improve performance above 40 MHz, a 32 KB single-cycle serial random access memory (SRAM), internal read-only memory (ROM) loaded with StellarisWare® software, 2KB electrically erasable programmable read-only memory (EEPROM), one or more pulse width modulation (PWM) modules, one or more quadrature encoder inputs (QEI) analog, one or more 12-bit Analog-to-Digital Converters (ADC) with 12 analog input channels, among other features that are readily available. Other microcontrollers may be readily substituted for use with the present disclosure. Accordingly, the present disclosure should not be limited in this context.

Signal communication can comprise any suitable form of communication in which information is transmitted between a sensor and the processor. Such communication can comprise wired communication utilizing one or more conductors and/or wireless communication utilizing a wireless transmitter and receiver. A surgical instrument can include a first sensor configured to detect a first condition of the surgical instrument and a second sensor configured to detect a second condition of the surgical instrument. For instance, the surgical instrument can include a first sensor configured to detect whether a closure trigger of the surgical instrument has been actuated and a second sensor configured to detect whether a firing trigger of the surgical instrument has been actuated .

Various embodiments are envisioned in which the surgical instrument can include two or more sensors configured to detect the same condition. In at least one such embodiment, the surgical instrument can comprise a processor, a first sensor in signal communication with the processor, and a second sensor in signal communication with the processor. The first sensor can be configured to communicate a first signal to the processor and the second sensor can be configured to communicate a second signal to the processor. In various instances, the processor can include a first input channel for receiving the first signal from the first sensor and a second input channel for receiving the second signal from the second sensor. In other instances, a multiplexer device can receive the first signal and the second signal and communicate the data of the first and second signals to the processor as part of a single, combined signal. In some instances, a first conductor, such as a first insulated wire can connect the first sensor to the first input channel and a second conductor, such as a second insulated wire can connect the second sensor to the second input channel. As outlined above, the first sensor and/or the second sensor can communicate wirelessly with the processor. In at least one such instance, the first sensor can include a first wireless transmitter and the second sensor can include a second wireless transmitter, wherein the processor can include and/or can be in communication with at least one wireless signal receiver configured to receive the first signal and/or the second signal and transmit the signals to the processor.

In co-operation with the sensors, as described in greater detail below, the processor of the surgical instrument can verify that the surgical instrument is operating correctly. The first signal can include data regarding a condition of the surgical instrument and the second signal can include data regarding the same condition. The processor can include an algorithm configured to compare the data from the first signal to the data from the second signal and determine whether the data communicated by the two signals are the same or different. If the data from the two signals are the same, the processor may use the data to operate the surgical instrument. In such circumstances, the processor can assume that a fault condition does not exist. In various instances, the processor can determine whether the data from the first signal and the data from the second signal are within an acceptable, or recognized, range of data. If the data from the two signals are within the recognized range of data, the processor may use the data from one or both of the signals to operate the surgical instrument. In such circumstances, the processor can assume that a fault condition does not exist. If the data from the first signal is outside of the recognized range of data, the processor may assume that a fault condition exists with regard to the first sensor, ignore the first signal, and operate the surgical instrument in response to the data from the second signal. Likewise, if the data from the second signal is outside the recognized range of data, the processor may assume that a fault condition exists with regard to the second sensor, ignore the second signal, and operate the surgical instrument in response to the data from the first signal. The processor can be configured to selectively ignore the input from one or more sensors.

The processor can include a module configured to implement an algorithm configured to assess whether the data from the first signal is between a first value and a second value. Similarly, the algorithm can be configured to assess whether the data from the second signal is between the first value and the second value. In certain instances, a surgical instrument can include at least one memory device. A memory device can be integral with the processor, in signal communication with the processor, and/or accessible by the processor. In certain instances, the memory device can include a memory chip including data stored thereon. The data stored on the memory chip can be in the form of a lookup table wherein the processor can access the lookup table to establish the acceptable, or recognized, range of data. In certain instances, the memory device can comprise nonvolatile memory, such as bit-masked read-only memory (ROM) or flash memory. Nonvolatile memory (NVM) may comprise other types of memory including programmable ROM (PROM), erasable programmable ROM (EPROM), electrically erasable programmable ROM (EEPROM), or battery backed random-access memory (RAM) such as dynamic RAM (DRAM), Double-Data-Rate DRAM (DDRAM), and/or synchronous DRAM (SDRAM).

Further to the above, the first sensor and the second sensor can be redundant. The processor can be configured to compare the first signal from the first sensor to the second signal from the second sensor to determine what action, if any, to take. In addition to or in lieu of the above, the processor can be configured to compare the data from the first signal and/or the second signal to limits established by the algorithm and/or data stored within a memory device. The processor can be configured to apply a gain to a signal it receives, such as the first signal and/or the second signal. For instance, the processor can apply a first gain to the first signal and a second gain to the second signal. In certain instances, the first gain can be the same as the second gain. In other instances, the first gain and the second gain can be different. In some circumstances, the processor can be configured to calibrate the first gain and/or the second gain. In at least one such circumstance, the processor can modify a gain such that the amplified signal is within a desired, or acceptable, range. In various instances, the unmodified gain and/or the modified gain can be stored within a memory device which is integral to and/or accessible by the processor. In certain embodiments, the memory device can track the history of the gains applied to a signal. In any event, the processor can be configured to provide this calibration before, during, and/or after a surgical procedure.

In various embodiments, the first sensor can apply a first gain to the first signal and the second sensor can apply a second gain to the second signal. In certain embodiments, the processor can include one or more output channels and can communicate with the first and second sensors. For instance, the processor can include a first output channel in signal communication with the first sensor and a second output channel in signal communication with the second sensor. Further to the above, the processor can be configured to calibrate the first sensor and/or the second sensor. The processor can send a first calibration signal through said first output channel in order to modify a first gain that the first sensor is applying to the first signal. Similarly, the processor can send a second calibration signal through said second output channel in order to modify a second gain that the second sensor is applying to the second signal.

As discussed above, the processor can modify the operation of the surgical instrument in view of the data received from the first signal and/or the second signal. In some circumstances, the processor can ignore the signal from a redundant sensor that the processor deems to be faulty. In some circumstances, the processor can return the surgical instrument to a safe state and/or warn the user of the surgical instrument that one or both of the sensors may be faulty. The processor can disable the surgical instrument. The processor can deactivate and/or modify certain functions of the surgical instrument when the processor detects that one or more of the sensors may be faulty. In at least one such circumstance, the processor may limit the operable controls to those controls which can permit the surgical instrument to be safely removed from the surgical site when the processor detects that one or more of the sensors may be faulty. In at least one circumstance, when the processor detects that one or more of the sensors may be faulty. The processor may limit the maximum speed, power, and/or torque that can be delivered by the motor of the surgical instrument when the processor detects that one or more of the sensors may be faulty. The processor may enable a recalibration control which may allow the user of the surgical instrument to recalibrate the mal-performing or non-performing sensor when the processor detects that one or more of the sensors may be faulty. While various exemplary embodiments utilizing two sensors to detect the same condition are described herein, various other embodiments are envisioned which utilize more than two sensors. The principles applied to the two sensor system described herein can be adapted to systems including three or more sensors.

As discussed above, the first sensor and the second sensor can be configured to detect the same condition of the surgical instrument. For instance, the first sensor and the second sensor can be configured to detect whether an anvil of the surgical instrument is in an open condition. In at least one such instance, the first sensor can detect the movement of a closure trigger into an actuated position and the second sensor can detect the movement of an anvil into a clamped position. In some instances, the first sensor and the second sensor can be configured to detect the position of a firing member configured to deploy staples from an end effector of the surgical instrument. In at least one such instance, the first sensor can be configured to detect the position of a motor-driven rack in a handle of the surgical instrument and the second sensor can be configured to detect the position of a firing member in a shaft or an end effector of the surgical instrument which is operably coupled with the motor-driven rack. In various instances, the first and second sensors could verify that the same event is occurring. The first and second sensors could be located in the same portion of the surgical instrument and/or in different portions of the surgical instrument. A first sensor can be located in the handle and a second sensor could be located in the shaft or the end effector.

Further to the above, the first and second sensors can be utilized to determine whether two events are occurring at the same time. For example, whether the closure trigger and the anvil are moving, or have moved, concurrently. In certain instances, the first and second sensors can be utilized to determine whether two events are not occurring at the same time. For example, it may not be desirable for the anvil of the end effector to open while the firing member of the surgical instrument is being advanced to deploy the staples from the end effector. The first sensor can be configured to determine whether the anvil is in an clamped position and the second sensor can be configured to determine whether the firing member is being advanced. In the event that the first sensor detects that the anvil is in an unclamped position while the second sensor detects that the firing member is being advanced, the processor can interrupt the supply of power to the motor of the surgical instrument. Similarly, the first sensor can be configured to detect whether an unclamping actuator configured to unclamp the end effector has been depressed and the second sensor can be configured to detect whether a firing actuator configured to operate the motor of the surgical instrument has been depressed. The processor of the surgical instrument can be configured to resolve these conflicting instructions by stopping the motor, reversing the motor to retract the firing member, and/or ignoring the instructions from the unclamping actuator.

In some instances, further to the above, the condition detected can include the power consumed by the surgical instrument. In at least one such instance, the first sensor can be configured to monitor the current drawn from a battery of the surgical instrument and the second sensor can be configured to monitor the voltage of the battery. As discussed above, such information can be communicated from the first sensor and the second sensor to the processor. With this information, the processor can calculate the electrical power draw of the surgical instrument. Such a system could be referred to as 'supply side' power monitoring. In certain instances, the first sensor can be configured to detect the current drawn by a motor of the surgical instrument and the second sensor can be configured to detect the current drawn by a processor of the surgical instrument, for example. As discussed above, such information can be communicated from the first sensor and the second sensor to the processor. With this information, the processor can calculate the electrical power draw of the surgical instrument. To the extent that other components of the surgical instrument draw electrical power, a sensor could be utilized to detect the current drawn for each component and communicate that information to the processor. Such a system could be referred to as 'use side' power monitoring. Various embodiments are envisioned which utilize supply side power monitoring and use side power monitoring. In various instances, the processor, and/or an algorithm implemented by the processor, can be configured to calculate a state of the device using more than one sensor that may not be sensed directly by only one sensor. Based on this calculation, the processor can enable, block, and/or modify a function of the surgical instrument.

The condition of the surgical instrument that can be detected by a processor and a sensor system can include the orientation of the surgical instrument. In at least one embodiment, the surgical instrument can include a handle, a shaft extending from the handle, and an end effector extending from the shaft. A first sensor can be positioned within the handle and a second sensor can be positioned within the shaft. The first sensor can comprise a first tilt sensor and the second sensor can comprise a second tilt sensor. The first tilt sensor can be configured to detect the instrument's orientation with respect to a first plane and the second tilt sensor can be configured to detect the instrument's orientation with respect to a second plane. The first plane and the second plane may or may not be orthogonal. The first sensor can comprise an accelerometer and/or a gyroscope. The second sensor can comprise an accelerometer and/or a gyroscope. Various embodiments are envisioned which comprise more than two sensors and each such sensor can comprise an accelerometer and/or a gyroscope. In at least one implementation, a first sensor can comprise a first accelerometer arranged along a first axis and a second sensor can comprise a second accelerometer arranged along a second axis which is different than the first axis. In at least one such instance, the first axis can be transverse to the second axis.

Further to the above, the processor can utilize data from the first and second accelerometers to determine the direction in which gravity is acting with respect to the instrument, i.e., the direction of ground with respect to the surgical instrument. In certain instances, magnetic fields generated in the environment surrounding the surgical instrument may affect one of the accelerometers. Further to the above, the processor can be configured to ignore data from an accelerometer if the data from the accelerometers is inconsistent. Moreover, the processor can be configured to ignore data from an accelerometer if the accelerometer is dithering between two or more strong polarity orientations. To the extent that an external magnetic field is affecting two or more, and/or all, of the accelerometers of a surgical instrument, the processor can deactivate certain functions of the surgical instrument which depend on data from the accelerometers. In various instances, a surgical instrument can include a screen configured to display images communicated to the screen by the processor, wherein the processor can be configured to change the orientation of the images displayed on the screen when the handle of the surgical instrument is reoriented, or at least when a reorientation of the handle is detected by the accelerometers. In at least one instance, the display on the screen can be flipped upside down when the handle is oriented upside down. In the event that the processor determines that orientation data from one or more of the accelerometers may be faulty, the processor may prevent the display from being reoriented away from its default position.

Further to the above, the orientation of a surgical instrument may or may not be detectable from a single sensor. In at least one instance, the handle of the surgical instrument can include a first sensor and the shaft can include a second sensor. Utilizing data from the first sensor and the second sensor, and/or data from any other sensor, the processor can determine the orientation of the surgical instrument. In some instances, the processor can utilize an algorithm configured to combine the data from the first sensor signal, the second sensor signal, and/or any suitable number of sensor signals to determine the orientation of the surgical instrument. In at least one instance, a handle sensor positioned within the handle can determine the orientation of the handle with respect to gravity. A shaft sensor positioned within the shaft can determine the orientation of the shaft with respect to gravity. In embodiments where the shaft, or at least a portion of the shaft, does not articulate relative to the handle, the processor can determine the direction in which the shaft, or the non-articulated shaft portion, is pointing. In some instances, a surgical instrument can include an end effector which can articulate relative to the shaft. The surgical instrument can include an articulation sensor which can determine the direction and the degree in which the end effector has been articulated relative to the shaft. With data from the handle sensor, the shaft sensor, and the articulation sensor, the processor can determine the direction in which the end effector is pointing. With additional data including the length of the handle, the shaft, and/or the end effector, the processor can determine the position of the distal tip of the end effector. With such information, the processor could enable, block, and/or modify a function of the surgical instrument.

In various instances, a surgical instrument can include a redundant processor in addition to a first processor. The redundant processor can be in signal communication with some or all of the sensors that the first processor is in signal communication with. The redundant processor can perform some or all of the same calculations that the first processor performs. The redundant processor can be in signal communication with the first processor. The first processor can be configured to compare the calculations that it has performed with the calculations that the redundant processor has performed. Similarly, the redundant processor can be configured to compare the calculations that it has performed with the calculations that the first processor has performed. In various instances, the first processor and the redundant processor can be configured to operate the surgical instrument independently of one another. In some instances, the first processor and/or the redundant processor can be configured to determine whether the other processor is faulty and/or deactivate the other processor if a fault within the other processor and/or within the surgical instrument is detected. The first processor and the redundant processor can both be configured to communicate with the operator of the surgical instrument such that, if one of the processors determines the other processor to be faulty, the non-faulty processor can communicate with the operator that a fault condition exists .

In various embodiments, a surgical instrument can include a processor and one or more sensors in signal communication with the processor. The sensors can comprise digital sensors and/or analog sensors. A digital sensor can generate a measuring signal and can include an electronic chip. The electronic chip can convert the measuring signal into a digital output signal. The digital output signal can then be transmitted to the processor utilizing a suitable transmission means such as a conductive cable, a fiber optic cable, and/or a wireless emitter. An analog sensor can generate a measuring signal and communicate the measuring signal to the processor using an analog output signal. An analog sensor can include a Hall Effect sensor, a magnetoresistive sensor, an optical sensor, and/or any other suitable sensor. A surgical instrument can include a signal filter which can be configured to receive and/or condition the analog output signal before the analog output signal reaches the processor. The signal filter can comprise a low-pass filter that passes signals to the processor having a low frequency which is at and/or below a cutoff frequency and that attenuates, or reduces the amplitude of, signals with high frequencies higher than the cutoff frequency. In some instances, the low-pass filter may eliminate certain high frequency signals that it receives or all of the high frequency signals that it receives. The low-pass filter may also attenuate, or reduce the amplitude of, certain or all of the low frequency signals, but such attenuation may be different than the attenuation that it applies to high frequency signals. Any suitable signal filter could be utilized. A high-pass filter could be utilized. A longpass filter could be utilized to receive and condition signals from optical sensors. In various instances, the processor can include an integral signal filter. In some instances, the processor can be in signal communication with the signal filter. In any event, the signal filter can be configured to reduce noise within the analog output signal, or signals, that it receives.

Further to the above, an analog output signal from a sensor can comprise a series of voltage potentials applied to an input channel of the processor. In various instances, the voltage potentials of the analog sensor output signal can be within a defined range. For instance, the voltage potentials can be between about 0V and about 12V, between about 0V and about 6V, between about 0V and about 3V, and/or between about 0V and about 1V. In some instances, the voltage potentials can be less than or equal to 12V, less than or equal to 6V, less than or equal to 3V, and/or less than or equal to 1V. In some instances, the voltage potentials can be between about 0V and about -12V, between about 0V and about -6V, between about 0V and about -3V, and/or between about 0V and about -1V. In some instances, the voltage potentials can be greater than or equal to -12V, greater than or equal to -6V, greater than or equal to -3V, and/or greater than or equal to -1V. In some instances, the voltage potentials can be between about 12V and about -12V, between about 6V and about -6V, between about 3V and about -3V, and/or between about 1V and about -1V. In various instances, the sensor can supply voltage potentials to an input channel of the processor in a continuous stream. The processor may sample this stream of data at a rate which is less than rate in which data is delivered to the processor. In some instances, the sensor can supply voltage potentials to an input channel of the process intermittently or at periodic intervals. In any event, the processor can be configured to evaluate the voltage potentials applied to the input channel or channels thereof and operate the surgical instrument in response to the voltage potentials, as described in greater detail further below.

Further to the above, the processor can be configured to evaluate the analog output signal from a sensor. In various instances, the processor can be configured to evaluate every voltage potential of the analog output signal and/or sample the analog output signal. When sampling the analog output signal, the processor can make periodic evaluations of the signal to periodically obtain voltage potentials from the analog output signal. For each evaluation, the processor can compare the voltage potential obtained from the evaluation against a reference value. The processor can calculate a digital value, such as 0 or 1, or on or off from this comparison. For instance, in the event that the evaluated voltage potential equals the reference value, the processor can calculate a digital value of 1. Alternatively, the processor can calculate a digital value of 0 if the evaluated voltage potential equals the reference value. With regard to a first embodiment, the processor can calculate a digital value of 1 if the evaluated voltage potential is less than the reference value and a digital value of 0 if the evaluated voltage potential is greater than the reference value. With regard to a second embodiment, the processor can calculate a digital value of 0 if the evaluated voltage potential is less than the reference value and a digital value of 1 if the evaluated voltage potential is greater than the reference value. In either event, the processor can convert the analog signal to a digital signal. When the processor is continuously evaluating the voltage potential of the sensor output signal, the processor can continuously compare the voltage potential to the reference value, and continuously calculate the digital value. When the processor is evaluating the voltage potential of the sensor output signal at periodic intervals, the processor can compare the voltage potential to the reference value at periodic intervals, and calculate the digital value at periodic intervals.

Further to the above, the reference value can be part of an algorithm utilized by the processor. The reference value can be pre-programmed in the algorithm. In some instances, the processor can obtain, calculate, and/or modify the reference value in the algorithm. In some instances, the reference value can be stored in a memory device which is accessible by and/or integral with the processor. The reference value can be pre-programmed in the memory device. In some instances, the processor can obtain, calculate, and/or modify the reference value in the memory device. In at least one instance, the reference value may be stored in non-volatile memory. In some instances, the reference value may be stored in volatile memory. The reference value may comprise a constant value. The reference value may or may not be changeable or overwritten. In certain instances, the reference value can be stored, changed, and/or otherwise determined as the result of a calibration procedure. The calibration procedure can be performed when manufacturing the surgical instrument, when initializing, or initially powering up, the instrument, when powering up the instrument from a sleep mode, when using the instrument, when placing the instrument into a sleep mode, and/or when completely powering down the instrument.

Also further to the above, the processor can be configured to store the digital value. The digital value can be stored at an electronic logic gate. In various instances, the electronic logic gate can supply a binary output which can be referenced by the processor to assess a condition detected by the sensor, as described in greater detail further below. The processor can include the electronic logic gate. The binary output of the electronic logic gate can be updated. In various instances, the processor can include one or more output channels. The processor can supply the binary output to at least one of the output channels. The processor can apply a low voltage to such an output channel to indicate an off bit or a high voltage to the output channel to indicate an on bit. The low voltage and the high voltage can be measured relative to a threshold value. In at least one instance, the low voltage can comprise no voltage. In at least one other instance, the low voltage can comprise a voltage having a first polarity and the high voltage can comprise a voltage having an opposite polarity.

In at least one instance, if the voltage potentials evaluated by the processor are consistently at or below the reference value, the electronic logic gate can maintain an output of 'on'. When an evaluated voltage potential exceeds the reference value, the output of the logic gate can be switched to 'off'. If the voltage potentials evaluated by the processor are consistently above the reference value, the electronic logic gate can maintain an output of 'off'. When an evaluated voltage potential is thereafter measured at or below the reference value, the output of the logic gate can be switched back to 'on', and so forth. In various instances, the electronic logic gate may not maintain a history of its output. In some instances, the processor can include a memory device configured to record the output history of the electronic logic gate, i.e., record a history of the calculated digital value. In various instances, the processor can be configured to access the memory device to ascertain the current digital value and/or at least one previously-existing digital value.

In various instances, the processor can provide an immediate response to a change in the calculated digital value. When the processor first detects that the calculated digital value has changed from 'on' to 'off' or from 'off' to 'on' the processor can immediately modify the operation of the surgical instrument. In certain instances, the processor may not immediately modify the operation of the surgical instrument upon detecting that the calculated digital value has changed from 'on' to 'off' or from 'off' to 'on'. The processor may employ a hysteresis algorithm. For instance, the processor may not modify the operation of the surgical instrument until after the digital value has been calculated the same way a certain number of consecutive times. In at least one such instance, the processor may calculate an 'on' value and display an 'on' binary value at the output logic gate and/or the output channel based on the data it has received from one or more surgical instrument sensors wherein, at some point thereafter, the processor may calculate an 'off' value based on the data it has received from one or more of the surgical instrument sensors; however, the processor may not immediately display an 'off' binary value at the output logic gate and/or the output channel. Rather, the processor may delay changing the binary value at the output logic gate and/or the output channel until after the processor has calculated the 'off' value a certain number of consecutive times, such as ten times. Once the processor has changed the binary value at the output logic gate and/or the output channel, the processor may likewise delay changing the binary value at the output logic gate and/or the output channel until after the processor has calculated the 'on' value a certain number of consecutive times, such as ten times and so forth.

A hysteresis algorithm may be suitable for handling switch debounce. A surgical instrument can include a switch debouncer circuit which utilizes a capacitor to filter out any quick changes of signal response.

In the example provided above, the sampling delay for going from 'on' to 'off' was the same as the sampling delay for going from 'off' to 'on'. Embodiments are envisioned in which the sampling delays are not equal. For instance, if an 'on' value at an output channel activates the motor of the surgical instrument and an 'off' value at an output channel deactivates the motor, the 'on' delay may be longer than the 'off' delay. In such instances, the processor may not suddenly activate the motor in response to accidental or incidental movements of the firing trigger while, on the other hand, the processor may react quickly to a release of the firing trigger to stop the motor. In at least one such instance, the processor may have an 'on' delay but no 'off' delay such that the motor can be stopped immediately after the firing trigger is released. As discussed above, the processor may wait for a certain number of consecutive consistent binary output calculations before changing the binary output value. Other algorithms are contemplated. For instance, a processor may not require a certain number of consecutive consistent binary output calculations; rather, the processor may only require that a certain number, or percentage, of consecutive calculations be consistent in order to change the binary output.

As discussed above, a processor can convert an analog input signal to a digital output signal utilizing a reference value. As also discussed above, the processor can utilize the reference value to convert the analog input data, or samples of the analog input data, to 'on' values or 'off' values as part of its digital output signal. In various instances, a processor can utilize more than one reference value in order to determine whether to output an 'on' value or an 'off' value. One reference value can define two ranges. A range below the reference value and a range above the reference value. The reference value itself can be part of the first range or the second range, depending on the circumstances. The use of additional reference values can define additional ranges. For instance, a first reference value and a second reference value can define three ranges: a first range below the first reference value, a second range between the first reference value and the second reference value, and a third range above the second reference value. Again, the first reference value can be part of the first range or the second range and, similarly, the second reference value can be part of the second range or the third range, depending on the circumstances. For a given sample of data from an analog signal, the processor can determine whether the sample lies within the first range, the second range, or the third range. In at least one exemplary embodiment, the processor can assign an 'on' value to the binary output if the sample is in the first range and an 'off' value to the binary output if the sample is in the third range. Alternatively, the processor can assign an 'off' value to the binary output if the sample is in the first range and an 'on' value to the binary output if the sample is in the third range.

Further to the above, the processor can assign an 'on' value or an 'off' value to the binary output if the data sample is in the second range. In various instances, an analog data sample in the second range may not change the binary output value. For instance, if the processor has been receiving analog data above the second reference value and producing a certain binary output and, subsequently, the processor receives analog data between the first reference value and the second reference value, the processor may not change the binary output. If the processor, in this example, receives analog data below the first reference value, the processor may then change the binary output. Correspondingly, in this example, if the processor has been receiving analog data below the first reference value and producing a certain binary output and, subsequently, the processor receives analog data between the first reference value and the second reference value, the processor may not change the binary output. If the processor, in this example, receives analog data above the second reference value, the processor may then change the binary output. In various instances, the second range between the first reference value and the second reference value may comprise an observation window within which the processor may not change the binary output signal. In certain instances, the processor may utilize different sampling delays, depending on whether the analog input data jumps directly between the first range and the third range or whether the analog input data transitions into the second range before transitioning into the third range. For example, the sampling delay may be shorter if the analog input data transitions into the second range before transitioning into the first range or the third range as compared to when analog input data jumps directly between the first range and the third range.

As discussed above, an analog sensor, such as a Hall effect sensor can be utilized to detect a condition of a surgical instrument. In various instances, a Hall effect sensor can produce a linear analog output which can include a positive polarity and a negative polarity and produce a wide range of analog output values. Such a wide range of values may not always be useful, or may not correspond to events which are actually possible for the surgical instrument. For instance, a Hall effect sensor can be utilized to track the orientation of the anvil of an end effector which, owing to certain physical constraints to the motion of the anvil, may only move through a small range of motion, such as about 30 degrees. Although the Hall effect sensor could detect motion of the anvil outside this range of motion, as a practical matter, the Hall effect sensor will not need to and, as a result, a portion of the output range of the Hall effect sensor may not be utilized. The processor may be programmed to only recognize a range of output from the Hall effect sensor which corresponds to a possible range of motion of the anvil and, to the extent that the processor receives data from the Hall effect sensor which is outside of this range of output, whether above the range or below the range, the processor can ignore such data, generate a fault condition, modify the operation of the surgical instrument, and/or notify the user of the surgical instrument. In such instances, the processor may recognize a valid range of data from the sensor and any data received from the sensor which is outside of this range may be deemed invalid by the processor. The valid range of data may be defined by a first reference value, or threshold, and a second reference value, or threshold. The valid range of data may include data having a positive polarity and a negative polarity. Alternatively, the valid range of data may only comprise data from the positive polarity or data from the negative polarity.

The first reference value and the second reference value, further to the above, can comprise fixed values. The first reference value and/or the second reference value can be calibrated. The first reference value and/or the second reference value can be calibrated when the surgical instrument is initially manufactured and/or subsequently re-manufactured. For instance, a trigger, such as the closure trigger can be moved through its entire range of motion during a calibration procedure and a Hall effect sensor positioned within the surgical instrument handle can detect the motion of the closure trigger, or at least the motion of a magnetic element, such as a permanent magnet positioned on the closure trigger. When the closure trigger is in its unclamped position, the reading taken by the Hall effect sensor can be stored as a first set point which corresponds with the unclamped position of the closure trigger. Similarly, when the closure trigger is in its fully clamped position, the reading taken by the Hall effect sensor can be stored as a second set point which corresponds with the fully clamped position of the closure trigger. Thereafter, the first set point can define the first reference value and the second set point can define the second reference value. Positions of the closure trigger between its unclamped position and its fully clamped position can correspond to the range of data between the first reference value and the second reference value. As outlined above, the processor can produce a digital output value in response to the data received from the analog sensor. In at least one instance, the processor can assign an 'off' value to its digital output when the data received from the analog sensor is at or above the first reference value. Alternatively, the processor can assign an 'off' value to its digital output when the data received from the analog sensor is above, at, or within about 20% of the range preceding first reference value. Data from the analog sensor which is between the first reference value and about 20% of the range below the first reference value can correspond with a position of the closure trigger which is suitably close to is unclamped position. In at least one instance, the processor can assign an 'on' value to its digital output when the data received from the analog sensor is below the first reference value. Alternatively, the processor can assign an 'on' value to its digital output when the data received from the analog sensor is at, below, or within about 40% of the range above the second reference value can correspond with a position of the closure trigger when it has been pulled about 3/4 through its range of motion. The same or similar attributes could be applied to a firing trigger of the surgical instrument.

Further to the above, a sensor can be calibrated in view of a reference value. For instance, if a reference value of+2V is associated with an unclamped position of the closure trigger and the processor detects a sensor output value which is different than +2V when the closure trigger is in its unclamped position, the processor can recalibrate the sensor, or the gain of the sensor, such that the sensor output matches, or at least substantially matches, the reference value. The processor may utilize an independent method of confirming that the closure trigger is in its unclamped position. In at least one such instance, the surgical instrument can include a second sensor in signal communication with the processor which can independently verify that the closure trigger is in its unclamped position. The second sensor could also comprise an analog sensor, such as a Hall effect sensor. The second sensor could comprise a proximity sensor, a resistance based sensor, and/or any other suitable sensor. The same or similar attributes could be applied to a firing trigger of the surgical instrument.

As discussed above, referring to FIGS. 14-18A, a tracking system 800 comprises one or more sensors, such as a first Hall effect sensor 803 and a second Hall effect sensor 804 which are configured to track the position of the magnet 802. Upon comparing FIGS. 14 and 17, the reader will appreciate that, when the closure trigger 32 is moved from its unactuated position to its actuated position, the magnet 802 moves between a first position adjacent the first Hall effect sensor 803 and a second position adjacent the second Hall effect sensor 804. When the magnet 802 is in its first position, the position of the magnet 802 is detected by the first Hall effect sensor 803 and/or the second Hall effect sensor 804. The processor of the surgical instrument uses data from the first sensor 803 to determine the position of the magnet 802 and data from the second sensor 804 to independently determine the position of the magnet 802. The processor utilizes data from the second sensor 804 to verify the integrity of the data from the first sensor 803. Alternatively, the processor could utilize the data from the first sensor 803 to verify the integrity of the data from the second sensor 804. The processor can utilize any suitable hierarchy for determining whether the data from a sensor should be used to provide a primary determination or a secondary determination of the position of the magnet 802. For instance, when the magnet 802 is in its first position, the magnet 802 provides a larger disturbance to the magnetic field surrounding the first sensor 803 than to the magnetic field surrounding the second sensor 804 and, as a result, the processor may utilize the data from the first sensor 803 as a primary determination of the position of the magnet 802. When the magnet 802 is closer to the second sensor 804 than the first sensor 803, the magnet 802 provides a larger disturbance to the magnetic field surrounding the second sensor 804 than to the magnetic field surrounding the first sensor 803 and, as a result, the processor may utilize the data from the second sensor 804 as a primary determination of the position of the magnet 802.

Further to the above, the path of the magnet 802 relative to the first sensor 803 can be determined when the magnet 802 moves along a first path segment when the closure trigger 32 is moved between its unclamped position and its clamped position and a second path segment when the firing trigger 130 is moved between its unfired position and its fired position. The range of outputs that the first sensor 803 will produce while tracking the magnet 802 as it moves along its first path segment can define a first valid range of data while the range of outputs that the first sensor 803 will produce while tracking the magnet 802 as it moves along its second path segment can define a second valid range of data. The first valid range of data may or may not be contiguous with the second valid range of data. In either event, the path of the magnet 802 relative to the second sensor 804 can also be determined when the magnet 802 moves along its first path segment and its second path segment. The range of outputs that the second sensor 804 will produce while tracking the magnet 802 as it moves along its first path segment can define a first valid range of data while the range of outputs that the second sensor 804 will produce while tracking the magnet 802 as it moves along its second path segment can define a second valid range of data. When the first sensor 803 and/or the second sensor 804 receives data outside of its respective first valid range of data and second valid range of data, the processor may assume that an error has occurred, modify the operation of the surgical instrument, and/or notify the operator of the surgical instrument. In certain instances, the processor can be configured to utilize data from the first sensor 803 and the second sensor 804 to determine whether the surgical instrument has been positioned within a strong external magnetic field which can affect the operation of the surgical instrument. For instance, the magnet 802 may move along a path such that the first sensor 803 and the second sensor 804 do not produce the same output at the same time and, in the event that first sensor 803 and the second sensor 804 produce the same output at the same time, the processor can determine that a fault condition exists.

In accordance with various embodiments, the surgical instruments described herein may comprise one or more processors (e.g., microprocessor, microcontroller) coupled to various sensors. In addition, to the processor(s), a storage (having operating logic) and communication interface, are coupled to each other.

The processor may be configured to execute the operating logic. The processor may be any one of a number of single or multi-core processors known in the art. The storage may comprise volatile and non-volatile storage media configured to store persistent and temporal (working) copy of the operating logic.

The operating logic may be configured to process the collected biometric associated with motion data of the user, as described above. The operating logic may be configured to perform the initial processing, and transmit the data to the computer hosting the application to determine and generate instructions. For these embodiments, the operating logic may be further configured to receive information from and provide feedback to a hosting computer. In alternate embodiments, the operating logic may be configured to assume a larger role in receiving information and determining the feedback. In either case, whether determined on its own or responsive to instructions from a hosting computer, the operating logic may be further configured to control and provide feedback to the user.

The operating logic may be implemented in instructions supported by the instruction set architecture (ISA) of the processor, or in higher level languages and compiled into the supported ISA. The operating logic may comprise one or more logic units or modules. The operating logic may be implemented in an object oriented manner. The operating logic may be configured to be executed in a multi-tasking and/or multi-thread manner. Alternatively or in addition, the operating logic may be implemented in hardware such as a gate array.

The communication interface may be configured to facilitate communication between a peripheral device and the computing system. The communication may include transmission of the collected biometric data associated with position, posture, and/or movement data of the user's body part(s) to a hosting computer, and transmission of data associated with the tactile feedback from the host computer to the peripheral device. The communication interface may be a wired or a wireless communication interface. An example of a wired communication interface may include, but is not limited to, a Universal Serial Bus (USB) interface. An example of a wireless communication interface may include, but is not limited to, a Bluetooth interface.

The processor may be packaged together with the operating logic. The processor may be packaged together with the operating logic to form a System in Package (SiP). The processor may be integrated on the same die with the operating logic. The processor may be packaged together with the operating logic to form a System on Chip (SoC).

Generally, software, program modules, and/or engines include any software element arranged to perform particular operations or implement particular abstract data types. Software, program modules, and/or engines can include routines, programs, objects, components, data structures and the like that perform particular tasks or implement particular abstract data types. An implementation of the software, program modules, and/or engines components and techniques may be stored on and/or transmitted across some form of computer-readable media. In this regard, computer-readable media can be any available medium or media useable to store information and accessible by a computing device. Some embodiments also may be practiced in distributed computing environments where operations are performed by one or more remote processing devices that are linked through a communications network. In a distributed computing environment, software, program modules, and/or engines may be located in both local and remote computer storage media including memory storage devices. A memory such as a random access memory (RAM) or other dynamic storage device may be employed for storing information and instructions to be executed by the processor. The memory also may be used for storing temporary variables or other intermediate information during execution of instructions to be executed by the processor.

Although some embodiments may be illustrated and described as comprising functional components, software, engines, and/or modules performing various operations, it can be appreciated that such components or modules may be implemented by one or more hardware components, software components, and/or combination thereof. The functional components, software, engines, and/or modules may be implemented by logic (e.g., instructions, data, and/or code) to be executed by a logic device (e.g., processor). Such logic may be stored internally or externally to a logic device on one or more types of computer-readable storage media. Alternatively or in addition, the functional components such as software, engines, and/or modules may be implemented by hardware elements that may include processors, microprocessors, circuits, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor device, chips, microchips, chip sets, and so forth.

Examples of software, engines, and/or modules may include software components, programs, applications, computer programs, application programs, system programs, machine programs, operating system software, middleware, firmware, software modules, routines, subroutines, functions, methods, procedures, software interfaces, application program interfaces (API), instruction sets, computing code, computer code, code segments, computer code segments, words, values, symbols, or any combination thereof. Determining whether an embodiment is implemented using hardware elements and/or software elements may vary in accordance with any number of factors, such as desired computational rate, power levels, heat tolerances, processing cycle budget, input data rates, output data rates, memory resources, data bus speeds and other design or performance constraints.

One or more of the modules described herein may comprise one or more embedded applications implemented as firmware, software, hardware, or any combination thereof. One or more of the modules described herein may comprise various executable modules such as software, programs, data, drivers, application program interfaces (APIs), and so forth. The firmware may be stored in a memory of the controller 2016 and/or the controller 2022 which may comprise a nonvolatile memory (NVM), such as in bit-masked read-only memory (ROM) or flash memory. In various implementations, storing the firmware in ROM may preserve flash memory. The nonvolatile memory (NVM) may comprise other types of memory including programmable ROM (PROM), erasable programmable ROM (EPROM), electrically erasable programmable ROM (EEPROM), or battery backed random-access memory (RAM) such as dynamic RAM (DRAM), Double-Data-Rate DRAM (DDRAM), and/or synchronous DRAM (SDRAM).

In some cases, various embodiments may be implemented as an article of manufacture. The article of manufacture may include a computer readable storage medium arranged to store logic, instructions and/or data for performing various operations of one or more embodiments. In various embodiments the article of manufacture may comprise a magnetic disk, optical disk, flash memory or firmware containing computer program instructions suitable for execution by a general purpose processor or application specific processor. The embodiments, however, are not limited in this context.

The functions of the various functional elements, logical blocks, modules, and circuits elements described in connection with the embodiments disclosed herein may be implemented in the general context of computer executable instructions, such as software, control modules, logic, and/or logic modules executed by the processing unit. Generally, software, control modules, logic, and/or logic modules comprise any software element arranged to perform particular operations. Software, control modules, logic, and/or logic modules can comprise routines, programs, objects, components, data structures and the like that perform particular tasks or implement particular abstract data types. An implementation of the software, control modules, logic, and/or logic modules and techniques may be stored on and/or transmitted across some form of computer-readable media. In this regard, computer-readable media can be any available medium or media useable to store information and accessible by a computing device. Some embodiments also may be practiced in distributed computing environments where operations are performed by one or more remote processing devices that are linked through a communications network. In a distributed computing environment, software, control modules, logic, and/or logic modules may be located in both local and remote computer storage media including memory storage devices.

Additionally, it is to be appreciated that the embodiments described herein illustrate example implementations, and that the functional elements, logical blocks, modules, and circuits elements may be implemented in various other ways which are consistent with the described embodiments.

Unless specifically stated otherwise, it may be appreciated that terms such as "processing," "computing," "calculating," "determining," or the like, refer to the action and/or processes of a computer or computing system, or similar electronic computing device, such as a general purpose processor, a DSP, ASIC, FPGA or other programmable logic device, discrete gate or transistor logic, discrete hardware components, or any combination thereof designed to perform the functions described herein that manipulates and/or transforms data represented as physical quantities (e.g., electronic) within registers and/or memories into other data similarly represented as physical quantities within the memories, registers or other such information storage, transmission or display devices.

It is worthy of note that some embodiments may be described using the expression "coupled" and "connected" along with their derivatives. These terms are not intended as synonyms for each other. For example, some embodiments may be described using the terms "connected" and/or "coupled" to indicate that two or more elements are in direct physical or electrical contact with each other. The term "coupled," however, also may mean that two or more elements are not in direct contact with each other, but yet still co-operate or interact with each other. With respect to software elements the term "coupled" may refer to interfaces, message interfaces, application program interface (API), exchanging messages, and so forth.

The disclosed embodiments have application in conventional endoscopic and open surgical instrumentation as well as application in robotic-assisted surgery.

Embodiments of the devices disclosed herein can be designed to be disposed of after a single use, or they can be designed to be used multiple times. Embodiments may, in either or both cases, be reconditioned for reuse after at least one use. Reconditioning may include any combination of the steps of disassembly of the device, followed by cleaning or replacement of particular pieces, and subsequent reassembly. In particular, embodiments of the device may be disassembled, and any number of the particular pieces or parts of the device may be selectively replaced or removed in any combination. Upon cleaning and/or replacement of particular parts, embodiments of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, embodiments described herein may be processed before surgery. First, a new or used instrument may be obtained and when necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a medical facility. A device also may be sterilized using any other technique known in the art, including but not limited to beta or gamma radiation, ethylene oxide, or steam.

One skilled in the art will recognize that the herein described components (e.g., operations), devices, objects, and the discussion accompanying them are used as examples for the sake of conceptual clarity and that various configuration modifications are contemplated. Consequently, as used herein, the specific exemplars set forth and the accompanying discussion are intended to be representative of their more general classes. In general, use of any specific exemplar is intended to be representative of its class, and the non-inclusion of specific components (e.g., operations), devices, and objects should not be taken limiting.

With respect to the use of substantially any plural and/or singular terms herein, those having skill in the art can translate from the plural to the singular and/or from the singular to the plural as is appropriate to the context and/or application. The various singular/plural permutations are not expressly set forth herein for sake of clarity.

The herein described subject matter sometimes illustrates different components contained within, or connected with, different other components. It is to be understood that such depicted architectures are merely examples and that in fact many other architectures may be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated also can be viewed as being "operably connected," or "operably coupled," to each other to achieve the desired functionality, and any two components capable of being so associated also can be viewed as being "operably couplable," to each other to achieve the desired functionality. Specific examples of operably couplable include but are not limited to physically mateable and/or physically interacting components, and/or wirelessly interactable, and/or wirelessly interacting components, and/or logically interacting, and/or logically interactable components.

Some aspects may be described using the expression "coupled" and "connected" along with their derivatives. It should be understood that these terms are not intended as synonyms for each other. For example, some aspects may be described using the term "connected" to indicate that two or more elements are in direct physical or electrical contact with each other. In another example, some aspects may be described using the term "coupled" to indicate that two or more elements are in direct physical or electrical contact. The term "coupled," however, also may mean that two or more elements are not in direct contact with each other, but yet still co-operate or interact with each other.

In some instances, one or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

While particular aspects of the present subject matter described herein have been shown and described, it will be apparent to those skilled in the art that, based upon the teachings herein, changes and modifications may be made without departing from the subject matter described herein. The claims submitted herewith define the overall scope.

## Claims

1. A surgical instrument (10), comprising:
a handle (14);
a movable input (32);
an analog sensor configured to detect the position of said movable input, wherein said analog sensor is configured to produce an analog signal comprising analog data; and
a microcontroller comprising an input channel, wherein said analog sensor is in signal communication with said input channel, wherein said microcontroller is configured to compare said analog data to a reference value, and wherein said microcontroller is configured to produce a digital signal in response to said comparison, wherein said microcontroller is configured to sample said analog data, and wherein said microcontroller is configured to generate a digital bit for each sample of said analog data, wherein said microcontroller is configured to generate an on bit if a sample is above said reference value, and wherein said microcontroller is configured to generate an off bit if a sample is below said reference value.

2. The surgical instrument of Claim 1, wherein said reference value comprises a first reference value, and wherein said microcontroller is configured to compare said analog data to a second reference value.

3. The surgical instrument of Claim 2, wherein said microcontroller is configured to generate an on bit if a sample is between said first reference value and said second reference value, wherein said microcontroller is configured to generate an off bit if a sample is below said first reference value, and wherein said microcontroller can be configured to generate a fault condition if a sample is above said second reference value.

4. The surgical instrument of Claim 3, wherein said microcontroller is configured to generate an on bit if a sample is between said first reference value and said second reference value, wherein said microcontroller is configured to generate an off bit if a sample is above said first reference value, and wherein said microcontroller can be configured to generate a fault condition if a sample is below said second reference value.

5. The surgical instrument of Claim 1, wherein said microcontroller is configured to sample said analog data, wherein said microcontroller comprises an output channel, wherein said microcontroller is configured to communicate said digital signal to said output channel, wherein said reference value comprises a first reference value, wherein said microcontroller is configured to compare said analog data to a second reference value, wherein said microcontroller is configured to change said digital signal if a sample is below said first reference value or above said second reference value, and wherein said microcontroller is configured to not change said digital signal if a sample is between said first reference value and said second reference value.

6. The surgical instrument of Claim 1, wherein said microcontroller is configured to sample said analog data, wherein said microcontroller comprises an output channel, wherein said microcontroller is configured to communicate said digital signal to said output channel, wherein said reference value comprises a first reference value, wherein said microcontroller is configured to compare said analog data to a second reference value, wherein said microcontroller is configured to supply an off bit to said output channel if a sample is less than said first reference value, wherein said microcontroller is configured to supply an on bit to said output channel if a sample is greater than said second reference value, and wherein said microcontroller is configured to not change said digital signal if a sample is between said first reference value and said second reference value.

7. The surgical instrument of Claim 1, wherein said analog sensor comprises a Hall effect sensor, wherein said movable input comprises a magnetic element, and wherein the movement of said magnetic element is detectable by said Hall effect sensor.

8. The surgical instrument of Claim 1, wherein said analog sensor is selected from the group consisting of a Hall effect sensor, a magnetoresistive sensor, and an optical sensor.

9. The surgical instrument of Claim 1, further comprising a shaft assembly attachable to said handle, wherein said shaft assembly comprises a movable jaw, and wherein said movable input comprises a closure trigger configured to move said movable jaw.

10. The surgical instrument of Claim 1, wherein said microcontroller is configured to adjust said reference value.

11. The surgical instrument of Claim 1, wherein said surgical instrument further comprises a memory device, and wherein said reference value is stored in said memory device.

12. The surgical instrument of Claim 1, wherein said microcontroller is operated by an algorithm, and wherein said reference value is stored in said algorithm.

13. The surgical instrument of Claim 1, further comprising a staple cartridge.

## Patentansprüche

1. Chirurgisches Instrument (10), umfassend:
einen Griff (14);
einen beweglichen Eingang (32);
einen Analogsensor, der zum Nachweisen der Position des beweglichen Eingangs ausgelegt ist, wobei der Analogsensor zum Erzeugen eines analoge Daten umfassenden analogen Signals ausgelegt ist;
und
einen Mikrocontroller, der einen Eingangskanal umfasst, wobei der Analogsensor mit dem Eingangskanal in Signalkommunikation steht, wobei der Mikrocontroller zum Vergleichen der analogen Daten mit einem Bezugswert ausgelegt ist und wobei der Mikrocontroller zum Erzeugen eines digitalen Signals als Reaktion auf den Vergleich ausgelegt ist, wobei der Mikrocontroller zum Abtasten der analogen Daten ausgelegt ist, und wobei der Mikrocontroller zum Erzeugen eines digitalen Bits für jede Abtastung der analogen Daten ausgelegt ist, wobei der Mikrocontroller zum Erzeugen eines On-Bits ausgelegt ist, wenn eine Abtastung über dem Bezugswert liegt, und wobei der Mikrocontroller zum Erzeugen eines Off-Bits ausgelegt ist, wenn eine Abtastung unter dem Bezugswert liegt.

2. Chirurgisches Instrument nach Anspruch 1, wobei der Bezugswert einen ersten Bezugswert umfasst, und wobei der Mikrocontroller zum Vergleichen der analogen Daten mit einem zweiten Bezugswert ausgelegt ist.

3. Chirurgisches Instrument nach Anspruch 2, wobei der Mikrocontroller zum Erzeugen eines On-Bits ausgelegt ist, wenn eine Abtastung zwischen dem ersten Bezugswert und dem zweiten Bezugswert liegt, wobei der Mikrocontroller zum Erzeugen eines Off-Bits ausgelegt ist, wenn eine Abtastung unter dem ersten Bezugswert liegt, und wobei der Mikrocontroller dazu ausgelegt sein kann, einen Fehlerzustand zu erzeugen, wenn eine Abtastung über dem zweiten Bezugswert liegt.

4. Chirurgisches Instrument nach Anspruch 3, wobei der Mikrocontroller zum Erzeugen eines On-Bits ausgelegt ist, wenn eine Abtastung zwischen dem ersten Bezugswert und dem zweiten Bezugswert liegt, wobei der Mikrocontroller zum Erzeugen eines Off-Bits ausgelegt ist, wenn eine Abtastung über dem ersten Bezugswert liegt, und wobei der Mikrocontroller dazu ausgelegt sein kann, einen Fehlerzustand zu erzeugen, wenn eine Abtastung unter dem zweiten Bezugswert liegt.

5. Chirurgisches Instrument nach Anspruch 1, wobei der Mikrocontroller zum Abtasten der analogen Daten ausgelegt ist, wobei der Mikrocontroller einen Ausgangskanal umfasst, wobei der Mikrocontroller dazu ausgelegt ist, das digitale Signal an den Ausgangskanal zu übertragen, wobei der Bezugswert einen ersten Bezugswert umfasst, wobei der Mikrocontroller zum Vergleichen der analogen Daten mit einem zweiten Bezugswert ausgelegt ist, wobei der Mikrocontroller dazu ausgelegt ist, das digitale Signal zu ändern, wenn eine Abtastung unter dem ersten Bezugswert oder über dem zweiten Bezugswert liegt, und wobei der Mikrocontroller dazu ausgelegt ist, das digitale Signal nicht zu ändern, wenn eine Abtastung zwischen dem ersten Bezugswert und dem zweiten Bezugswert liegt.

6. Chirurgisches Instrument nach Anspruch 1, wobei der Mikrocontroller zum Abtasten der analogen Daten ausgelegt ist, wobei der Mikrocontroller einen Ausgangskanal umfasst, wobei der Mikrocontroller dazu ausgelegt ist, das digitale Signal an den Ausgangskanal zu übertragen, wobei der Bezugswert einen ersten Bezugswert umfasst, wobei der Mikrocontroller zum Vergleichen der analogen Daten mit einem zweiten Bezugswert ausgelegt ist, wobei der Mikrocontroller dazu ausgelegt ist, dem Ausgangskanal ein Off-Bit zuzuführen, wenn eine Abtastung kleiner als der erste Bezugswert ist, wobei der Mikrocontroller dazu ausgelegt ist, dem Ausgangskanal ein On-Bit zuzuführen, wenn eine Abtastung größer als der zweite Bezugswert ist, und wobei der Mikrocontroller dazu ausgelegt ist, das digitale Signal nicht zu ändern, wenn eine Abtastung zwischen dem ersten Bezugswert und dem zweiten Bezugswert liegt.

7. Chirurgisches Instrument nach Anspruch 1, wobei der Analogsensor einen Hall-Effekt-Sensor umfasst, wobei der bewegliche Eingang ein magnetisches Element umfasst, und wobei die Bewegung des magnetischen Elements vom Hall-Effekt-Sensor nachweisbar ist.

8. Chirurgisches Instrument nach Anspruch 1, wobei der Analogsensor aus der aus einem Hall-Effekt-Sensor, einem magnetoresistiven Sensor und einem optischen Sensor bestehenden Gruppe ausgewählt ist.

9. Chirurgisches Instrument nach Anspruch 1, ferner umfassend eine Schaftanordnung, die am Griff befestigbar ist, wobei die Schaftanordnung eine bewegliche Backe umfasst und wobei der bewegliche Eingang einen Schließauslöser umfasst, der zum Bewegen der beweglichen Backe ausgelegt ist.

10. Chirurgisches Instrument nach Anspruch 1, wobei der Mikrocontroller zum Anpassen des Bezugswerts ausgelegt ist.

11. Chirurgisches Instrument nach Anspruch 1, wobei das chirurgische Instrument ferner eine Speichervorrichtung umfasst und wobei der Bezugswert in der Speichervorrichtung gespeichert ist.

12. Chirurgisches Instrument nach Anspruch 1, wobei der Mikrocontroller von einem Algorithmus bedient wird, und wobei der Bezugswert in dem Algorithmus gespeichert ist.

13. Chirurgisches Instrument nach Anspruch 1, ferner umfassend ein Klammermagazin.

## Revendications

1. Instrument chirurgical (10), comprenant :
une poignée (14) ;
une entrée mobile (32) ;
un capteur analogique configuré pour détecter la position de ladite entrée mobile, ledit capteur analogique étant configuré pour produire un signal analogique comprenant des données analogiques ; et
un microcontrôleur comprenant un canal d'entrée, ledit capteur analogique étant en communication de signal avec ledit canal d'entrée, ledit microcontrôleur étant configuré pour comparer lesdites données analogiques à une valeur de référence, et ledit microcontrôleur étant configuré pour produire un signal numérique en réponse à ladite comparaison, ledit microcontrôleur étant configuré pour échantillonner lesdites données analogiques, et ledit microcontrôleur étant configuré pour générer un bit numérique pour chaque échantillon desdites données analogiques, ledit microcontrôleur étant configuré pour générer un bit ON si un échantillon est supérieur à ladite valeur de référence, et ledit microcontrôleur étant configuré pour générer un bit OFF si un échantillon est inférieur à ladite valeur de référence.

2. Instrument chirurgical selon la revendication 1, ladite valeur de référence comprenant une première valeur de référence, et ledit microcontrôleur étant configuré pour comparer lesdites données analogiques à une seconde valeur de référence.

3. Instrument chirurgical selon la revendication 2, ledit microcontrôleur étant configuré pour générer un bit ON si un échantillon se situe entre ladite première valeur de référence et ladite seconde valeur de référence, ledit microcontrôleur étant configuré pour générer un bit OFF si un échantillon est inférieur à ladite première valeur de référence, et ledit microcontrôleur pouvant être configuré pour générer une condition de défaut si un échantillon est supérieur à ladite seconde valeur de référence.

4. Instrument chirurgical selon la revendication 3, ledit microcontrôleur étant configuré pour générer un bit ON si un échantillon est entre ladite première valeur de référence et ladite seconde valeur de référence, ledit microcontrôleur étant configuré pour générer un bit OFF si un échantillon est supérieur à ladite première valeur de référence, et ledit microcontrôleur pouvant être configuré pour générer une condition de défaut si un échantillon est inférieur à ladite seconde valeur de référence.

5. Instrument chirurgical selon la revendication 1, ledit microcontrôleur étant configuré pour échantillonner lesdites données analogiques, ledit microcontrôleur comprenant un canal de sortie, ledit microcontrôleur étant configuré pour communiquer ledit signal numérique audit canal de sortie, ladite valeur de référence comprenant une première valeur de référence, ledit microcontrôleur étant configuré pour comparer lesdites données analogiques à une seconde valeur de référence, ledit microcontrôleur étant configuré pour modifier ledit signal numérique si un échantillon est inférieur à ladite première valeur de référence ou supérieur à ladite seconde valeur de référence, et ledit microcontrôleur étant configuré pour ne pas modifier ledit signal numérique si un échantillon est entre ladite première valeur de référence et ladite seconde valeur de référence.

6. Instrument chirurgical selon la revendication 1, ledit microcontrôleur étant configuré pour échantillonner lesdites données analogiques, ledit microcontrôleur comprenant un canal de sortie, ledit microcontrôleur étant configuré pour communiquer ledit signal numérique audit canal de sortie, ladite valeur de référence comprenant une première valeur de référence, ledit microcontrôleur étant configuré pour comparer lesdites données analogiques à une seconde valeur de référence, ledit microcontrôleur étant configuré pour fournir un bit OFF audit canal de sortie si un échantillon est inférieur à ladite première valeur de référence, ledit microcontrôleur étant configuré pour fournir un bit ON audit canal de sortie si un échantillon est supérieur à ladite seconde valeur de référence, et ledit microcontrôleur étant configuré pour ne pas modifier ledit signal numérique si un échantillon est entre ladite première valeur de référence et ladite seconde valeur de référence.

7. Instrument chirurgical selon la revendication 1, ledit capteur analogique comprenant un capteur à effet Hall, ladite entrée mobile comprenant un élément magnétique, et le mouvement dudit élément magnétique pouvant être détecté par ledit capteur à effet Hall.

8. Instrument chirurgical selon la revendication 1, ledit capteur analogique étant choisi dans le groupe constitué par un capteur à effet Hall, un capteur magnétorésistif et un capteur optique.

9. Instrument chirurgical selon la revendication 1, comprenant en outre un ensemble arbre pouvant être fixé à ladite poignée, ledit ensemble arbre comprenant une mâchoire mobile, et ladite entrée mobile comprenant un déclencheur de fermeture configuré pour déplacer ladite mâchoire mobile.

10. Instrument chirurgical selon la revendication 1, ledit microcontrôleur étant configuré pour ajuster ladite valeur de référence.

11. Instrument chirurgical selon la revendication 1, ledit instrument chirurgical comprenant en outre un dispositif de mémoire, et ladite valeur de référence étant stockée dans ledit dispositif de mémoire.

12. Instrument chirurgical selon la revendication 1, ledit microcontrôleur étant commandé par un algorithme, et ladite valeur de référence étant stockée dans ledit algorithme.

13. Instrument chirurgical selon la revendication 1, comprenant en outre une cartouche d'agrafes.
